# EUROPEAN PATENT APPLICATION

(11) **EP 1 914 256 A1**
(43) Date of publication of application: **23.04.2008**
(21) Application number: 06780991.3
(22) Date of filing: 12.07.2006
(51) Int. Cl.: C08G 59/20, C07D 303/48, C08G 65/04

(54) **COMPOSITION, ACTINIC ENERGY RADIATION HARDENABLE COMPOSITION AND EPOXY COMPOUND**

(30) Priority: 09.08.2005 JP 2005230695
(71) Applicant: Konica Minolta Medical & Graphic, Inc., Hino-shi, Tokyo 191-8511 (JP)
(72) Inventor: SASA, Nobumasa, Tokyo 1630512 (JP)
(74) Representative: Alton, Andrew
(86) International application number: PCT/JP2006/313821
(87) International publication number: WO 2007/018011

(57) **Abstract**

The invention is to provide an epoxy compound or an actinic energy radiation hardenable composition each having high safety and stability, and to provide an actinic energy radiation hardenable composition with excellent photo-hardenability under high humidity, which gives high solvent resistance, high water proof and a hardened layer with high strength. There is provided an actinic energy radiation hardenable composition containing an epoxy compound represented by the following formula (1).

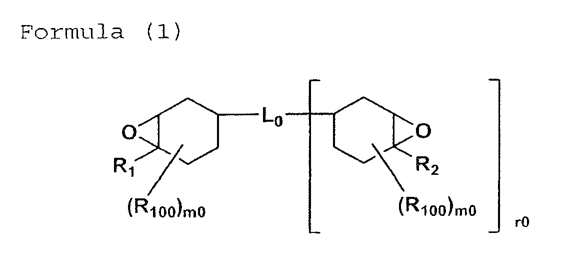

## Description

### FIELD OF THE INVENTION

The present invention relates to an actinic energy radiation hardenable composition, and particularly to an actinic energy radiation hardenable composition employed in printing ink; coatings for cans, plastics, papers or wood materials; adhesives or optical solid shaped forms.

### BACKGROUND OF THE INVENTION

In recent years, epoxy compounds, especially alicyclic epoxy compounds are widely employed together with a cationic photopolymerization initiator in an actinic energy radiation hardenable composition. For example, the epoxy compounds are employed in printing ink (see Patent Document 1), in coatings (see Patent Document 2 or 3), in coating for coating an outer surface of cans (see Patent Document 4), in coating for coating plastics (see Patent Document 5), in coating for coating papers (see Patent Document 6), in coating for coating wood materials (see Patent Document 7), in adhesives (see Patent Document 8), in optical solid shaping forms papers (see Patent Document 9 or 10), and in ink-jet ink (see Patent Document 11).

However, the epoxy compound disclosed in these patent documents or the actinic energy radiation hardenable composition containing the same disclosed in these patent documents have problems in safety, and the actinic energy radiation hardenable composition has problems in stability, hardenability (hardenability especially under high humidity condition), and layer strength, solvent resistance or water resistance of hardened layer obtained thereof.
Patent Document 1: JP-A No. 8-143806
Patent Document 2: JP-A No. 8-20627
Patent Document 3: JP-A No. 10-158581
Patent Document 4: JP-A No. 8-134405
Patent Document 5: JP-A No. 8-208832
Patent Document 6: JP-A No. 8-218296
Patent Document 7: JP-A No. 8-239623
Patent Document 8: JP-A No. 8-231938
Patent Document 9: JP-A No. 8-20728
Patent Document 10: JP-A No. 2000-62030
Patent Document 11: JP-A No.2004-315778

### DISCLOSURE OF THE INVENTION

### PROBLEMS TO BE SOLVED BY THR INVENTION

The present invention has been made in view of the above. An object of the invention is to provide an epoxy compound or an actinic energy radiation hardenable composition each having high safety and stability, and to provide an actinic energy radiation hardenable composition with excellent photo-hardenability under high humidity, which gives high solvent resistance, high water proof and a hardened layer with high strength.

### MEANS FOR SOLVING THE ABOVE PROBLEMS

The above object can be attained by one of the following constitutions:
1. A composition characterized in that it contains an epoxy compound represented by the following formula (1), 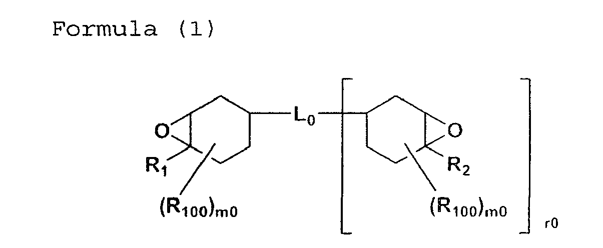 wherein R₁ and R₂ independently represent a first substituent (other than a hydrogen atom); R₁₀₀ represents a second substituent; m0 represents an integer of from 0 to 2; r0 represents an integer of from 1 to 3; and L₀ represents a simple bond or a (r0+1)-valent linkage group with a carbon atom number of from 1 to 15 containing in the main chain an oxygen atom and a nitrogen atom.
2. The composition of item 1 characterized in that the epoxy compound is represented by the following formula (2), 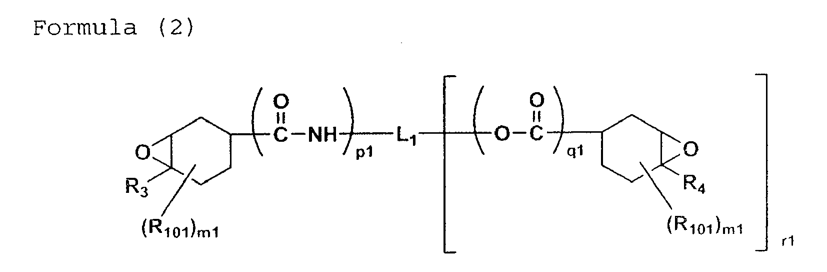 wherein R₃ and R₄ independently represent a third substituent (other than a hydrogen atom); R₁₀₁ represents a fourth substituent; m1 represents an integer of from 0 to 2; p1 represents an integer of from 1 to 2; q1 represents an integer of 0, 1 or 2; r1 represents an integer of 1 to 2; and L₁ represents a simple bond or a (r1+1)-valent linkage group with a carbon atom number of from 1 to 15 containing in the main chain an oxygen atom or a sulfur atom.
3. An actinic energy radiation hardenable composition characterized in that it contains an epoxy compound represented by the following formula (1), 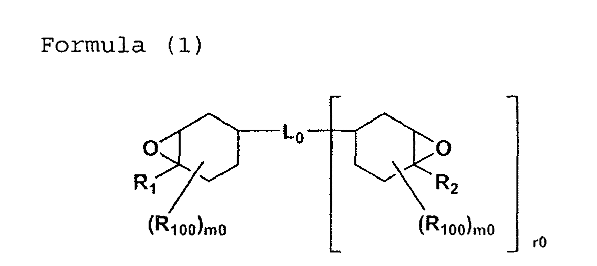 wherein R₁ and R₂ independently represent a first substituent (other than a hydrogen atom); R₁₀₀ represents a second substituent; m0 represents an integer of from 0 to 2; r0 represents an integer of from 1 to 3; and L₀ represents a simple bond or a (ro+1)-valent linkage group with a carbon atom number of from 1 to 15 containing in the main chain an oxygen atom and a nitrogen atom.
4. The actinic energy radiation hardenable composition of item 3 characterized in that the epoxy compound is represented by the following formula (2), 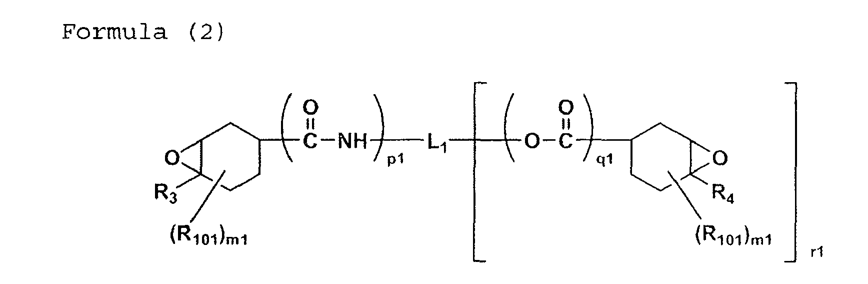 wherein R₃ and R₄ independently represent a third substituent (other than a hydrogen atom); R₁₀₁ represents a fourth substituent; m1 represents an integer of from 0 to 2; p1 represents an integer of from 1 to 2; q1 represents an integer of 0, 1 or 2; r1 represents an integer of from 1 to 2; and L₁ represents a simple bond or a (r1+1)-valent linkage group with a carbon atom number of from 1 to 15 containing in the main chain an oxygen atom or a sulfur atom.
5. The actinic energy radiation hardenable composition of item 3 or 4 characterized in that the epoxy compound has a molecular weight of from 170 to 1000.
6. The actinic energy radiation hardenable composition of any one of items 3 through 5 characterized in that it further contains an oxetane compound or a vinyl ether compound.
7. The actinic energy radiation hardenable composition of any one of items 3 through 6 characterized in that it further contains a cationic photopolymerization initiator.
8. The actinic energy radiation hardenable composition of any one of items 3 through 7 characterized in that it further contains, as a polymerizable compound, an oxetane compound and further contains, as a cationic photopolymerization initiator, at least one selected from sulfonium salts producing no benzene on actinic ray exposure represented by the formulae (4) through (7), 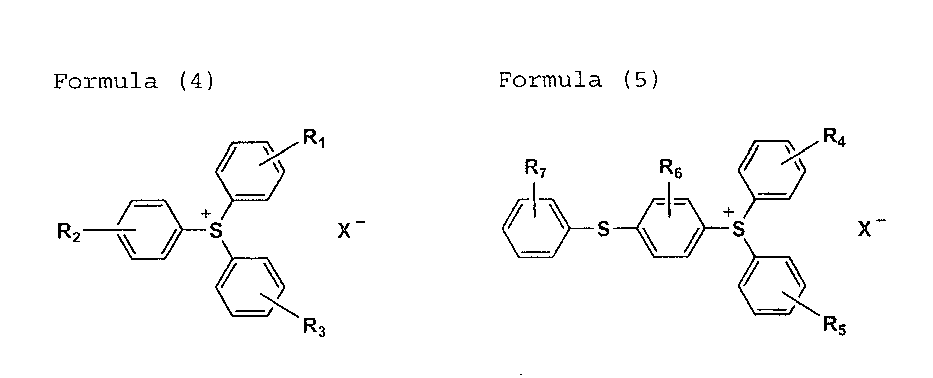 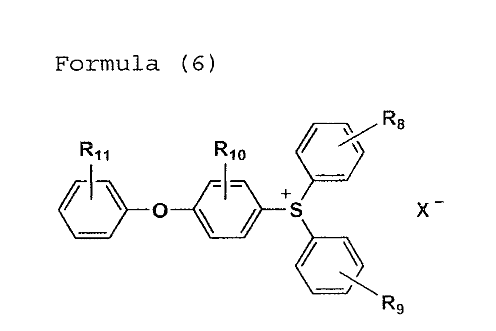 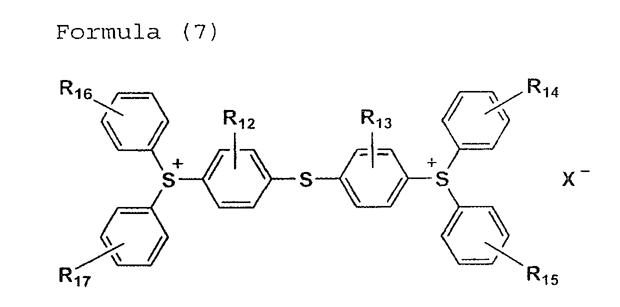 wherein R₁ through R₁₇ independently represent a hydrogen atom or a substituent, provided that R₁ through R₃ are not simultaneously hydrogens, R₄ through R₇ are not simultaneously hydrogens, R₈ through R₁₁ are not simultaneously hydrogens, and R₁₂ through R₁₇ are not simultaneously hydrogens; and X represents a non-nucleophilic anion.
9. The actinic energy radiation hardenable composition of item 8 characterized in that the sulfonium salts represented by the formulae (4) through (7) include a sulfonium salt selected from the sulfonium salts represented by the formulae (8) through (16), 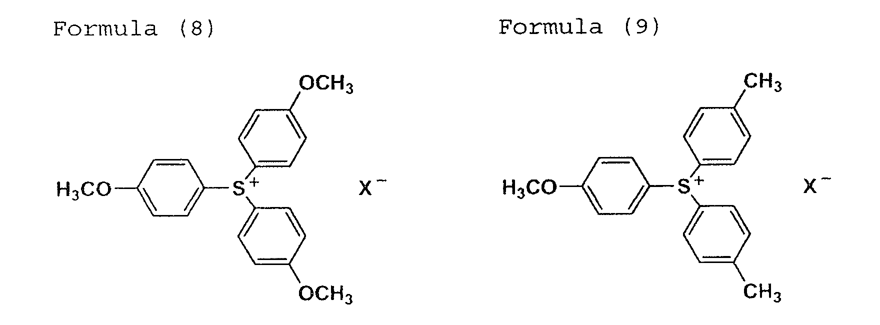 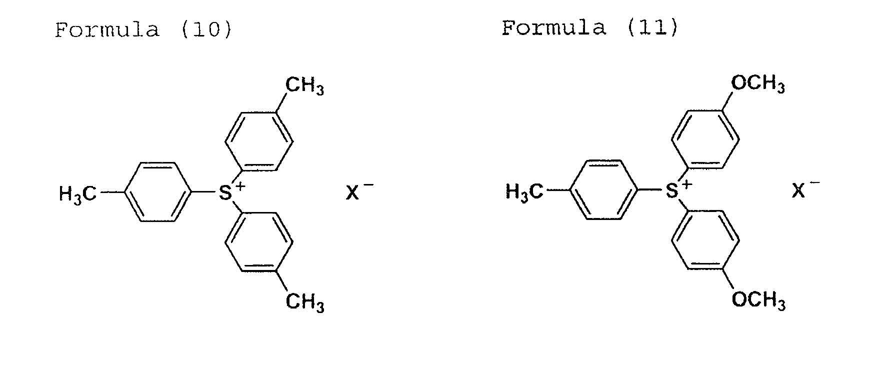 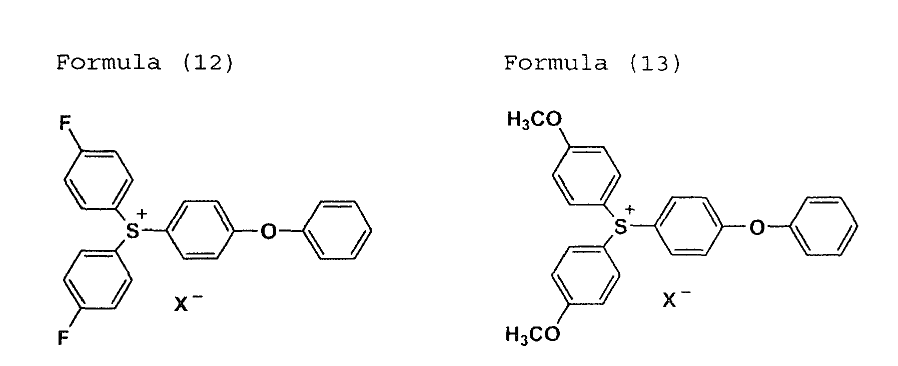 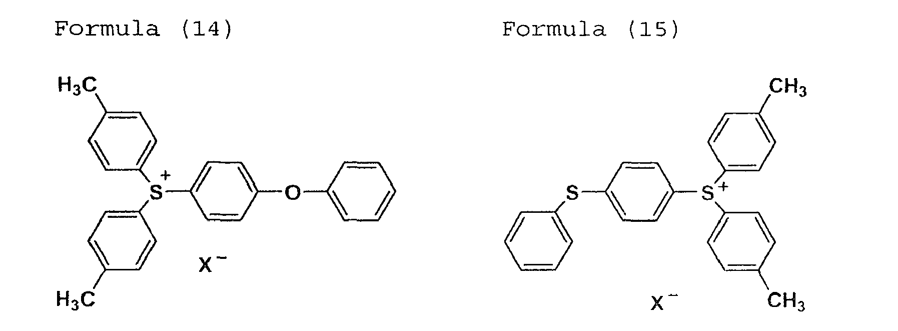 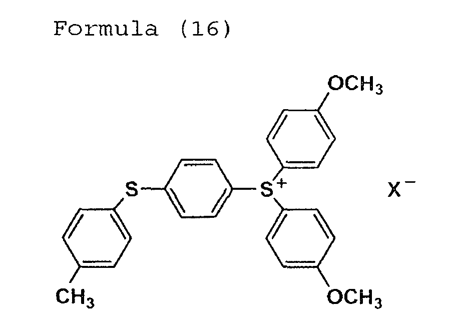 wherein x represents a non-nucleophilic anion.
10. The actinic energy radiation hardenable composition of any one of items 3 through 9 characterized in that it further contains pigment.
11. An epoxy compound represented by formula (1) above.
12. An epoxy compound represented by formula (2) above.
13. The epoxy compound of item 11 or 12 characterized in that a value obtained by dividing the molecular weight with the total number of epoxy groups in the molecule is from 160 to 300.

### EFFECT OF THE INVENTION

The present invention has been made in view of the above. An object of the invention is to provide an epoxy compound or an actinic energy radiation hardenable composition each having high safety and stability, and to provide an actinic energy radiation hardenable composition with excellent photo-hardenability under high humidity, which gives high solvent resistance, high water proof and a hardened layer with high strength.

### BRIEF DESCRIPTION OF DRAWINGS

Fig. 1 is an explanatory drawing for showing a process forming an unhardened composition layer which is not hardened in an optical solid shaping system.
Fig. 2 is an explanatory drawing for showing a process forming a first hardened layer in an optical solid shaping system.
Fig. 3 is an explanatory drawing for showing a process forming an unhardened composition layer on the first hardened layer in an optical solid shaping system.
Fig. 4 is an explanatory drawing for showing a process forming a second hardened layer in an optical solid shaping system.

### EXPLANATION OF NUMERICAL NUMBERS

- 1.: Control section
- 2.: NC table
- 3.: Optical system
- 4.: Laser
- 5.: Resin
- 6.: Laser beams
- 7.: First hardened layer
- 8.: Second hardened layer

### PREFERRED EMBODIMENT OF THE INVENTION

The preferred embodiment of the invention will be explained below, but the invention is not limited thereto.

The present invention will be explained in detail below.

The present invention relates to an actinic energy radiation hardenable composition characterized in that it contains an epoxy compound having a specific chemical structure or a sulfonium salt having a specific chemical structure. It is preferred in obtaining advantages of the invention more efficiently that the composition further contains an oxetane compound, a vinyl ether compound, a cationic photopolymerization initiator or pigment.

### (Epoxy compound)

Next, an epoxy compound represented by formula (1) or (2) used in the invention will be explained.

In formula above, R₁, R₂, R₃, R₄, R₁₀₀, and R₁₀₁ represent a substituent. Examples of the substituent include an alkyl group having a carbon atom number of from 1 to 6 (for example, a methyl group, an ethyl group, a propyl group, an isopropyl group, or a butyl group); an alkoxy group having a carbon atom number of from 1 to 6 (for example a methoxy group, an ethoxy group, an n-propoxy group, an iso-propoxy group, an n-butoxy group, or a tert-butoxy group); an acyl group (foe example, an acetyl group, a propionyl group or a trifluoroacetyl group); an acyloxy group (for example, an acetoxy group, a propionyloxy group or a trifluoroacetyloxy group); and an alkoxycarbonyl group (for example, a methoxycarbonyl group, an ethoxycarbonyl group or a tert-butoxycarbonyl group). The preferred substituents are an alkyl group, an alkoxy group and an alkoxycarbonyl group.

m0 and m1 represent an integer of from 0 to 2, and are preferably 0 or 1.

L₀ represents a simple bond or a (r0+1)-valent linkage group with a carbon atom number of from 1 to 15 containing in the main chain an oxygen atom and a nitrogen atom. L₁ represents a simple bond or a (rl+1)-valent linkage group with a carbon atom number of from 1 to 15 containing in the main chain an oxygen atom or a sulfur atom.

Herein, as the divalent linkage groups with a carbon number of from 1 to 15 which contain an oxygen atom or a sulfur atom in the main chain, are cited the following groups and their combination with -O-, -S-, -CO- and/or -CS-.
a methylene group [-CH₂-],
an ethylidene group [>CHCH₃],
an isopropylidene group [>C(CH₃)₂],
a 1,2-ethylene group [-CH₂CH₂-],
a 1,2-propylene group [-CH(CH₃)CH₂-],
a 1,3-propanediyl group [-CH₂CH₂CH₂-],
a 2,2-dimethyl-1,3-propanediyl group [-CH₂C(CH₃)₂CH₂-],
a 2,2-dimethoxy-1,3-propanediyl group [-CH₂C(OCH₃)₂CH₂-],
a 2,2-dimethoxymethyl-1,3-propanediyl group
[-CH₂C(CH₂OCH₃)₂CH₂-],
a 1-methyl-1, 3-propanediyl group [-CH(CH₃)CH₂CH₂-],
a 1,4-butanediyl group [-CH₂CH₂CH₂CH₂-],
a 1,5-pentanediyl group [-CH₂CH₂CH₂CH₂CH₂-],
an oxydiethylene group [-CH₂CH₂OCH₂CH₂-],
a thiodiethylene group [-CH₂CH₂SCH₂CH₂-],
a 3-oxothiodiethylene group [-CH₂CH₂SOCH₂CH₂-],
a 3, 3-dioxothiodiethylene group [-CH₂CH₂SO₂CH₂CH₂-]
a 1,4-dimethyl-3-oxa-1,5-pentanediyl group
[-CH(CH₃)CH₂CH₂OCH(CH₃)CH₂-],
a 3-oxopentanediyl group [-CH₂CH₂COCH₂CH₂-],
a 1,5-dioxo-3-oxapentanediyl group [-COCH₂OCH₂CO-],
a 4-oxa-1,7-heptanediyl group [-CH₂CH₂CH₂OCH₂CH₂CH₂-],
a 3, 6-dioxa-1, 8-octanediyl group [-CH₂CH₂OCH₂CH₂OCH₂CH₂-],
a 1,4,7-trimethyl-3,6-dioxa-1,8-octanediyl group
[-CH(CH₃)CH₂CH₂OCH(CH₃)CH₂CHOCH(CH₃)CH₂-],
a 5,5-dimethyl-3,7-dioxa-1,9-nonanediyl group
[-CH₂CH₂OCH₂CH₂C(CH₃)₂CH₂CH₂OCH₂CH₂-],
a 5,5-dimethoxy-3,7-dioxa-1,9-nonanediyl group
[-CH₂CH₂OCH₂CH₂C(OCH₃)₂CH₂CH₂OCH₂CH₂-],
a 5,5-dimethoxymethyl-3,7-dioxa-1,9-nonanediyl group
[-CH₂CH₂OCH₂CH₂C(CH₂OCH₃)₂CH₂CH₂OCH₂CH₂-],
a 4,7-dioxo-3,8-dioxa-1,10-decanediyl group
[-CH₂CH₂O-COCH₂CH₂CO-OCH₂CH₂],
a 3,8-dioxo-4,7-dioxa-1,10-decanediyl group
[-CH₂CH₂CO-OCH₂CH₂O-COCH₂CH₂-],
a 1,3-cyclopentanediyl group [-1,3-C₅H₈-],
a 1,2-cyclohexanediyl group [-1,2-C₆H₁₀-],
a 1,3-cyclohexanediyl group [-1,3-C₆H₁₀-],
a 1,4-cyclohexanediyl group [-1,4-C₆H₁₀-],
a 2,5-tetrahydrofurandiyl group [-2,5-C₄H₆O-],
a p-phenylene group [-p-C₆H₄-],
an m-phenylene group [-m-C₆H₄-],
an α,α' -o-xylylene group [-o-CH₂-C₆H₄-CH₂-],
an α,α' -m-xylylene group [-m-CH₂-C₆H₄-CH₂-],
an α,α' -p-xylylene group [-p-CH₂C₆H₄-CH₂-],
a furan-2, 5-diyl-bismethylene group [-2,5-CH₂-C₄H₂O-CH₂-],
a thiophene-2,5-diyl-bismethylene group [-2,5-CH₂-C₄H₂S-CH₂-],
an isopropylidene-p-phenylene group [-p-C₆H₄-C(CH₃)₂-p-C₆H₄-]

As tri- or more-valent linkage groups are cited groups in which an arbitrary hydrogen atom is withdrawn from the divalent linkage groups described above and their combination with -O-, -S-, -CO- and/or -CS-.

L₀ and L₁ may have a substituent. Examples of the substituent include a halogen atom (for example, a chlorine atom, a bromine atom, or a fluorine atom), an alkyl group having a carbon atom number of from 1 to 6 (for example, a methyl group, an ethyl group, a propyl group, an isopropyl group, or a butyl group), an alkoxy group having a carbon atom number of from 1 to 6 (for example, a methoxy group, an ethoxy group, an n-propoxy group, an isopropoxy group, a n-butoxy group or a tert-butoxy group), an acyl group (for example, an acetyl group, a propionyl group, or a trifluoroacetyl group), an acyloxy group (for example, an acetoxy group, a propionyloxy group, or a trifluoroacetoxy group), and an alkoxycarbonyl group (for example, a methoxycarbonyl group, an ethoxycarbonyl group, or a tert-butoxycarbonyl group). Preferred substituents are a halogen atom, an alkyl group, and an alkoxy group. R₄₀₅ and the substituent of L₁ and R₄₀₅ may combine with each other to form a ring.

L₀ is preferably a divalent linkage group with a carbon atom number of from 1 to 8 containing in the main chain an oxygen atom and a nitrogen atom, and more preferably one containing an amide bond in the main chain.

L₁ is preferably a divalent linkage group with a carbon atom number of from 1 to 8 containing in the main chain an oxygen atom or a sulfur atom, and more preferably a divalent linkage group with a carbon atom number of from 1 to 5 containing in the main chain only a carbon atom.

p1 represents an integer of from 1 to 2, and q1 represents an integer of from 0 to 2. It is preferred that (p1+q1) is 1 or more.

Preferred examples of the epoxy compound will be listed, but the invention is not limited thereto.

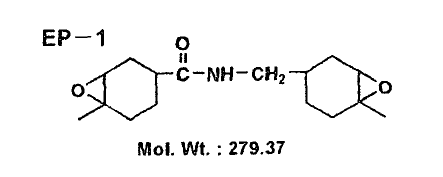

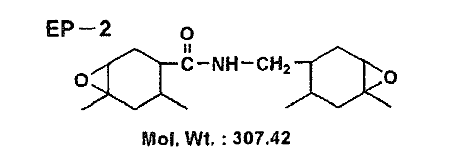

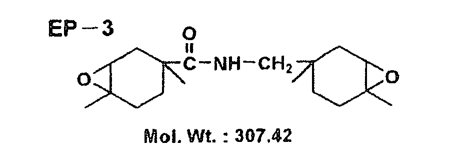

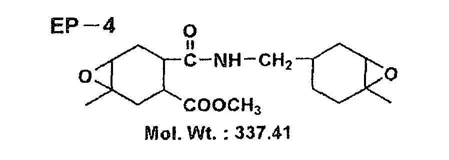

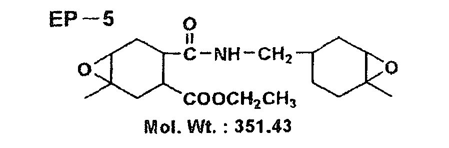

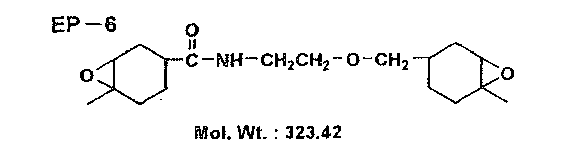

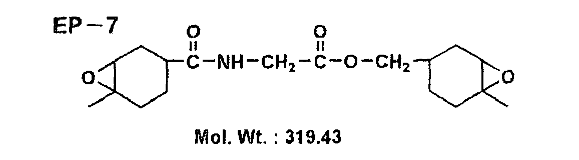

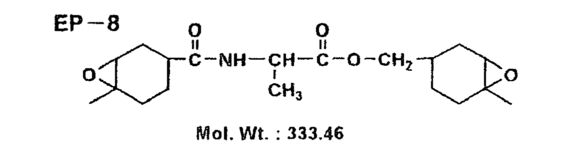

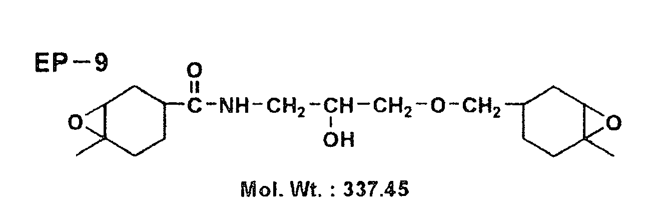

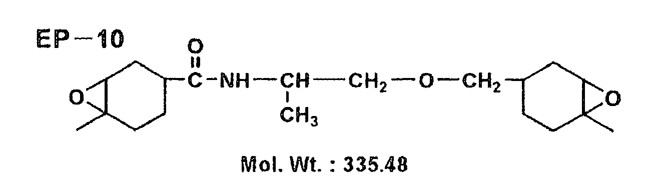

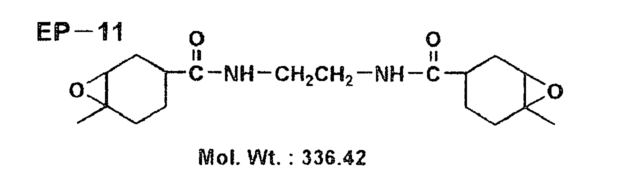

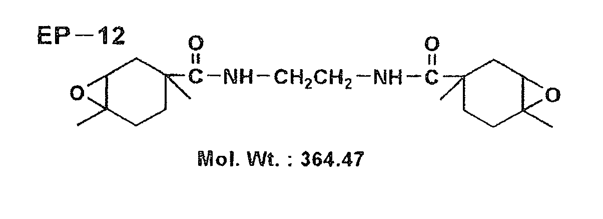

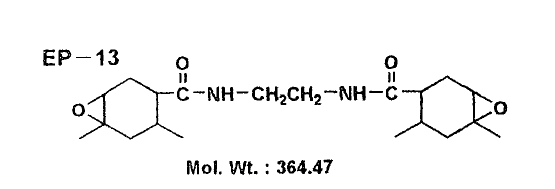

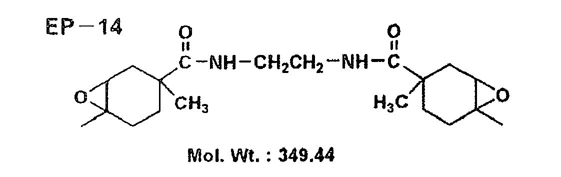

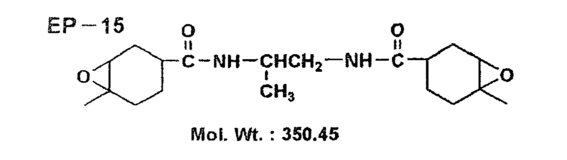

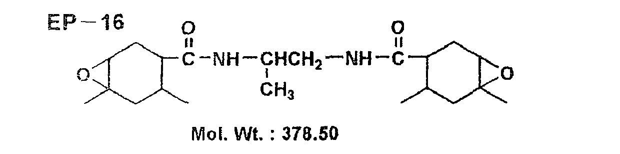

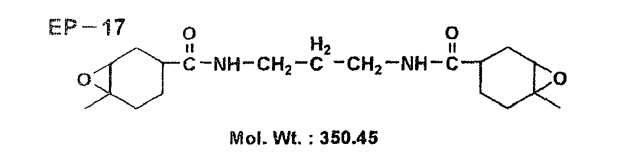

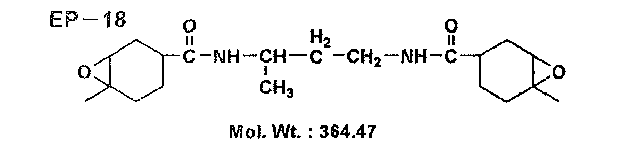

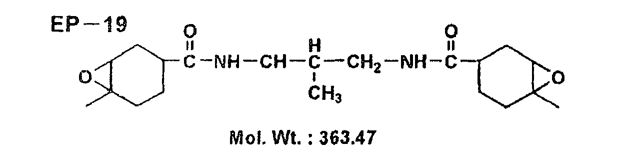

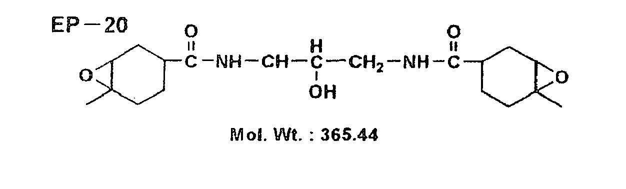

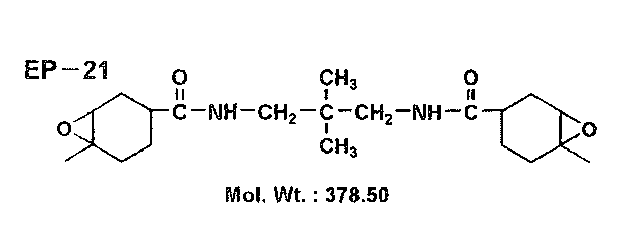

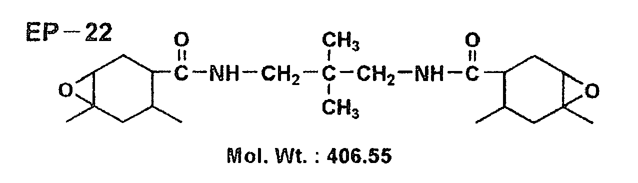

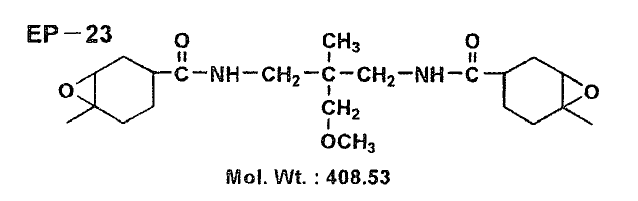

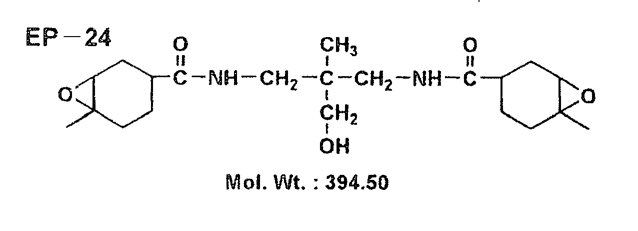

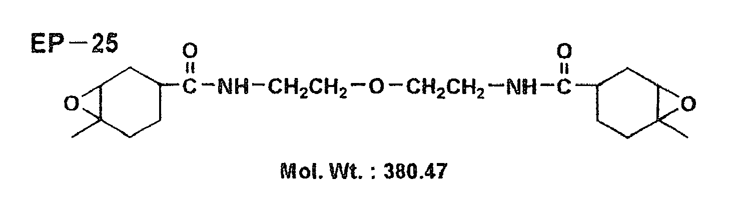

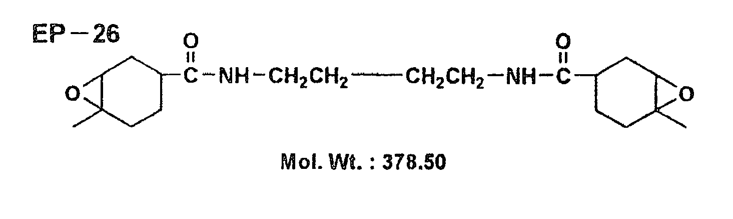

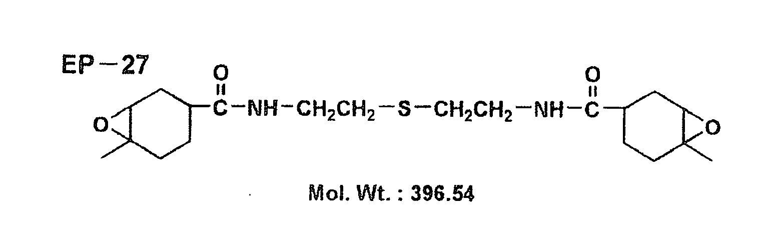

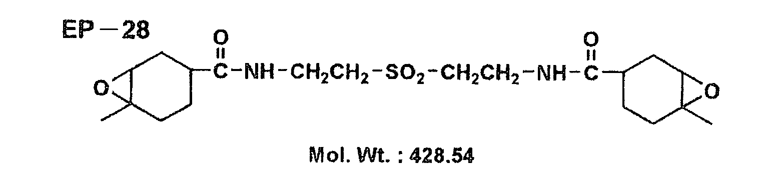

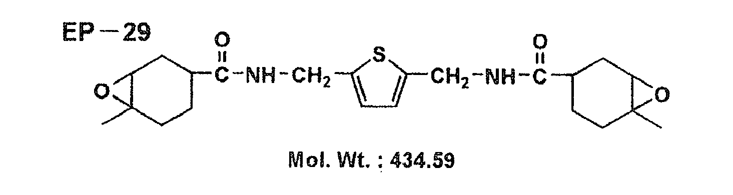

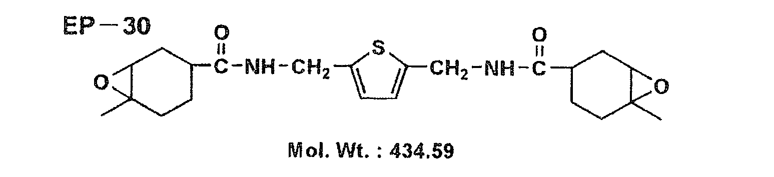

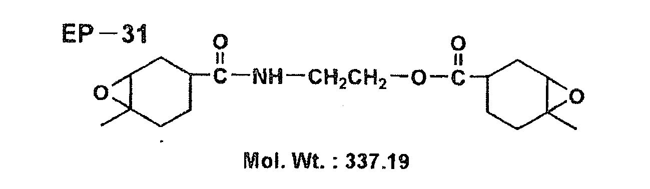

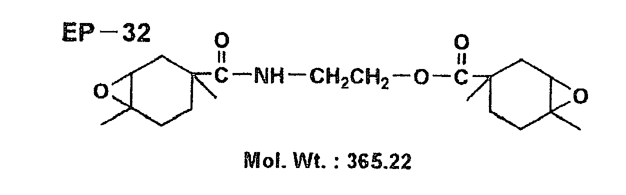

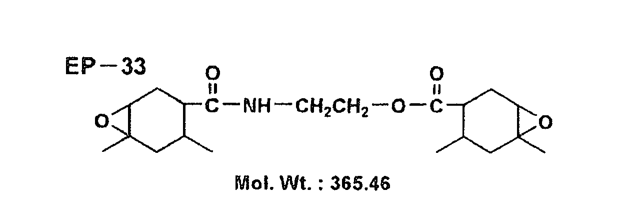

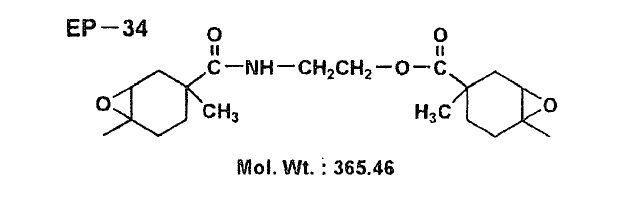

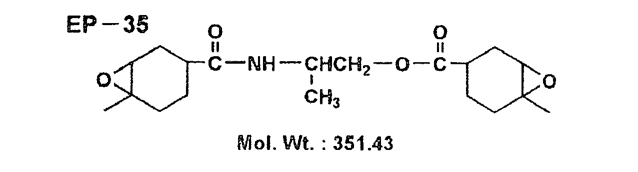

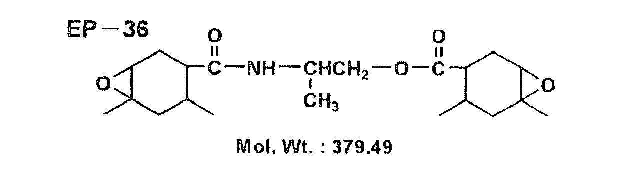

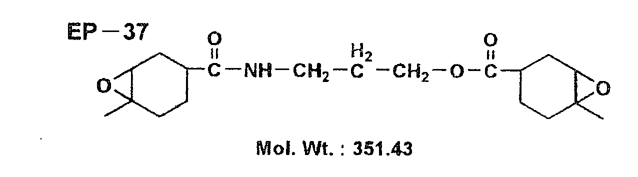

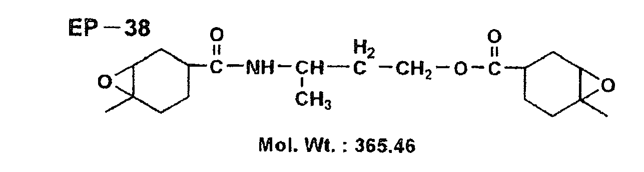

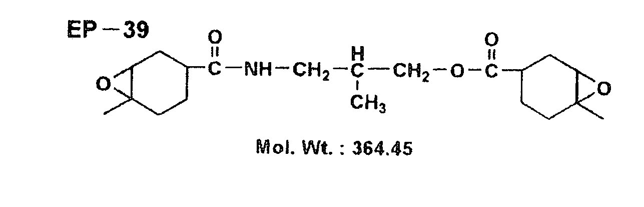

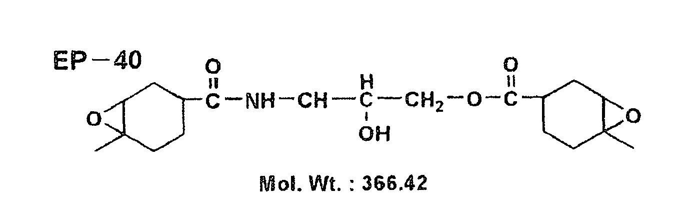

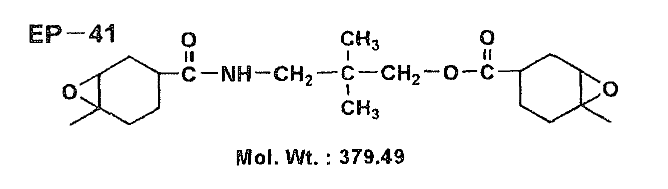

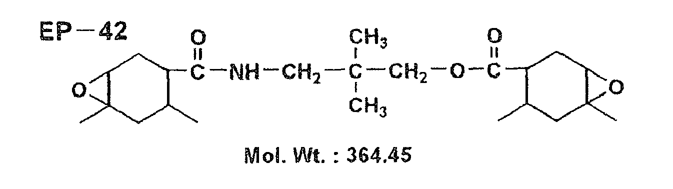

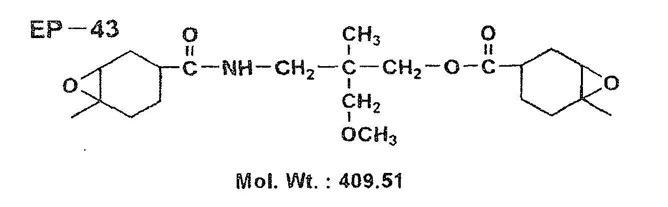

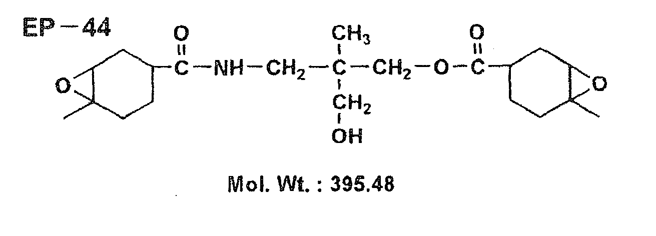

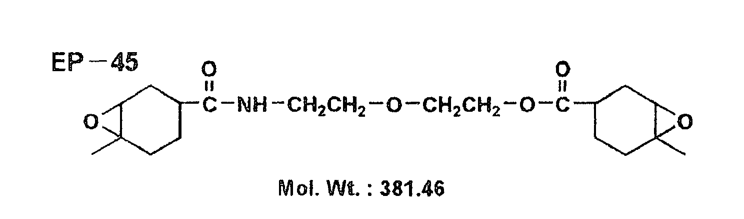

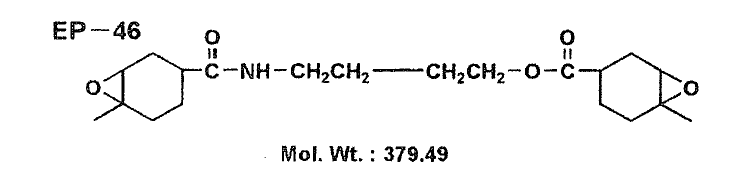

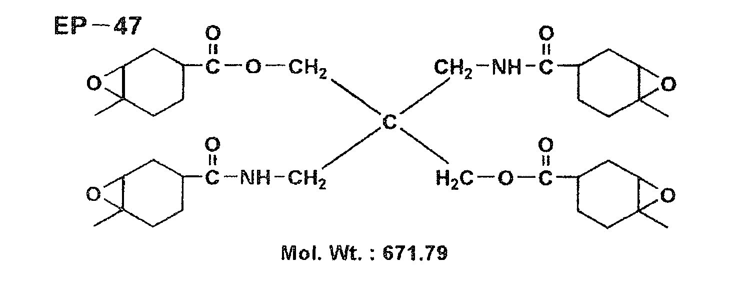

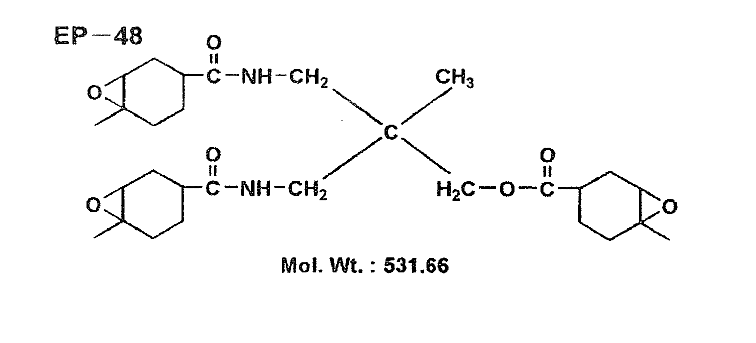

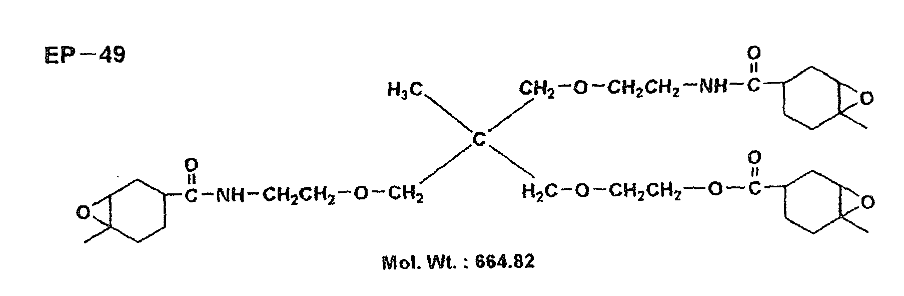

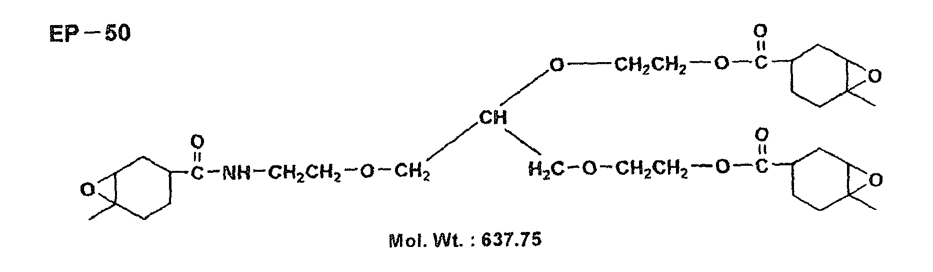

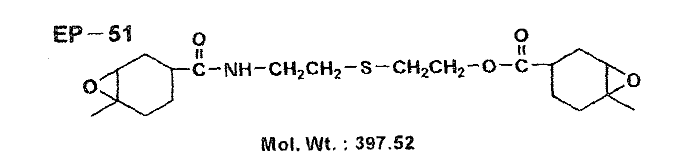

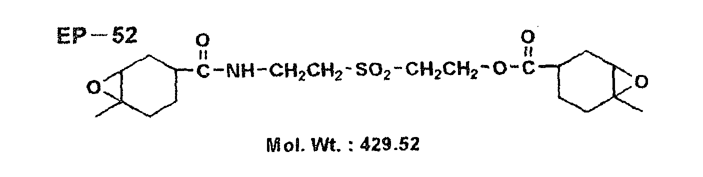

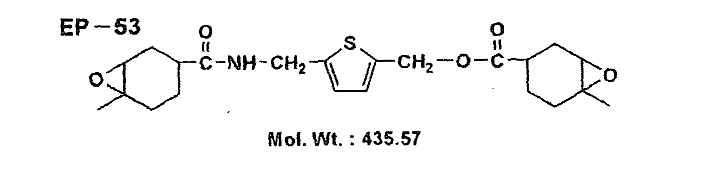

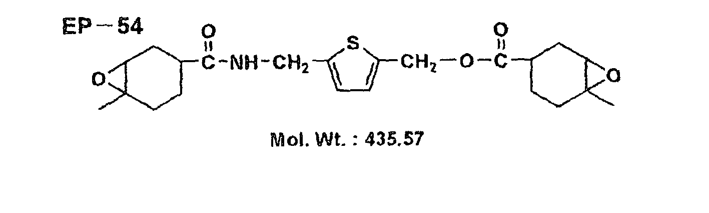

The epoxy compound of the invention can be synthesized in accordance with methods disclosed in patent documents, for example, in U.S. Patent Nos. 2,745,847, 2,750,395, 2,853,498, 2,853,499 and 2,863,881.

According to the methods disclosed in the foregoing patent documents, synthesis examples of the above exemplified compounds will be shown below, but the invention are not limited to these.

### Synthetic example 1

### Synthesis of Exemplified Compound EP-11 (Ethylenediamine-bis-(4-methyl-3,4-epoxy-cyclohexanecarboxylate)

### [Synthesis of methyl-(4-methyl-3-cyclohexenecarboxylate)]

Using isoprene and methyl acrylate as raw material, methyl-(4-methyl-3-cyclohexanecarboxylate) was synthesized through commonly known Diels-Alder reaction. The reaction was undergone under the reaction conditions described in J. Organomet. Chem., 285, 1985, 333-342; and J. Phys. Chem., 95, 5, 1992, 2293-2297; Acta. Chem. Scand. 47, 6, 1993, 581-591; U.S. Patent No. 1,944,731. Thus, an objective compound was obtained at a high yield.

### [Synthesis of Ethylenediamine-bis-(4-methyl-3-cyclohexenecarboxylate)]

To a mixture of 308 g (2 moles) of methyl-(4-methyl-3-cyclohexenecarboxylate) and 60 g (1 mole) of ethylenediamine was added 1 g of phosphoric acid, and the resulting mixture was reacted at 110 to 120 °C for 8 hrs. The reaction mixture was washed with an aqueous sodium bicarbonate solution and subjected to vacuum distillation to obtain an objective compound. The yield was 94%.

Into 2 lit. three-necked flask were introduced 304 g (1 mole) of ethylenediamine-bis-(4-methyl-3-cyclohexenecarboxylate), and further thereto, 770 g of an acetone solution having a peracetic acid content of 25% by weight [containing 192 g (2.5 mol) of peracetic acid] was dropwise added over a period of 4 hrs., while maintaining the internal temperature at 35 to 40 °C. After completion of addition, the reaction continued further for 4 hrs. at the same temperature. The reaction mixture was allowed to stand for one night at -11 °C and then, the residual amount of peracetic acid was checked and it was confirmed that at least 98% of the theoretical amount was reacted.

Subsequently, the reaction mixture was diluted with 1 lit. of toluene and heated at 50 °C under reduced pressure using a water-jet aspirator to distil away low boiling components until no distillate was formed. The remained reaction composition was subjected to vacuum distillation to obtain an objective compound. The yield was 82%.

### Synthetic example 2

### Synthesis of Exemplified compound EP-15 (Propane-1,2-diamine-bis-(4-methyl-3,4-epoxy-cyclohexanecarboxylate)

### [Synthesis of Propane-1,2-diamine-bis-(4-methyl-3-cyclohexenecarboxylate)]

To a mixture of 308 g (2 moles) of methyl-(4-methyl-3-cyclohexenecarboxylate) and 74 g (1 mole) of propane-1,2-diamine was added 1 g of phosphoric acid, and the resulting mixture was reacted at 110 to 120 °C for 8 hrs. The reaction mixture was washed with an aqueous sodium bicarbonate solution and subjected to vacuum distillation to obtain an objective compound. The yield was 92%.

Into 2 lit. three-necked flask were introduced 314 g (1 mole) of propane-1,2-diamine-bis-(4-methyl-3-cyclohexenecarboxylate), and further thereto, 770 g of an acetone solution having a peracetic acid content of 25% by weight [containing 192 g (2.5 mol) of peracetic acid] was dropwise added over a period of 4 hrs., while maintaining the internal temperature at 35 to 40 °C. After completion of addition, the reaction continued further for 4 hrs. at the same temperature. The reaction mixture was allowed to stand for one night at -11 °C and then, the residual amount of peracetic acid was checked and it was confirmed that at least 98% of the theoretical amount was reacted.

Subsequently, the reaction mixture was diluted with 1 lit. of toluene and heated at 50 °C under reduced pressure using a water-jet aspirator to distil away low boiling components until no distillate was formed. The remained reaction composition was subjected to vacuum distillation to obtain an objective compound. The yield was 78%.

### Synthetic example 3

### Synthesis of Exemplified compound EP-21 (2,2-Dimethyl-1-propane-1,3-diamine-bis-(4-methyl-3,4-epoxycyclohexanecarboxylate)

### [Synthesis of 2,2-Dimethyl-1-propane-1,3-diamine-bis-(4-methyl-3-cyclohexenecarboxylate)]

To a mixture of 308 g (2 moles) of methyl-(4-methyl-3-cyclohexenecarboxylate) and 102 g (1 mole) of 2,2-dimethylpropane-1,3-diamine was added 1 g of phosphoric acid, and the resulting mixture was reacted at 110 to 120 °C for 12 hrs. The reaction mixture was washed with an aqueous sodium bicarbonate solution and subjected to vacuum distillation to obtain an objective compound. The yield was 86%.

Into 2 lit. three-necked flask were introduced 348 g (1 mole) of 2,2-dimethyl-1-propane-1,3-diamine-bis-(4-methyl-3-cyclohexenecarboxylate), and further thereto, 770 g of an acetone solution having a peracetic acid content of 25% by weight [containing 192 g (2.5 mol) of peracetic acid] was dropwise added over a period of 4 hrs., while maintaining the internal temperature at 35 to 40 °C. After completion of addition, the reaction continued further for 4 hrs. at the same temperature. The reaction mixture was allowed to stand for one night at -11°C and then, the residual amount of peracetic acid was checked and it was confirmed that at least 98% of the theoretical amount was reacted.

Subsequently, the reaction mixture was diluted with 1 lit. of toluene and heated at 50 °C under reduced pressure using a water-jet aspirator to distil away low boiling components until no distillate was formed. The remained reaction composition was subjected to vacuum distillation to obtain an objective compound. The yield was 72%.

### Synthetic example 4

### Synthesis of Exemplified compound EP-31 (2-amino-ethanol-bis-(4-methyl-3,4-epoxy-cyclohexanecarboxylate)

### [Synthesis of 2-amino-ethanol-bis-(4-methyl-3,4-epoxy-cyclohexenecarboxylate)]

To a mixture of 308 g (2 moles) of methyl-(4-methyl-3-cyclohexenecarboxylate) and 61 g (1 mole) of 2-amino-ethanol was added 1 g of phosphoric acid, and the resulting mixture was reacted at 110 to 120 °C for 12 hrs. The reaction mixture was washed with an aqueous sodium bicarbonate solution and subjected to vacuum distillation to obtain an objective compound. The yield was 87%.

Into 2 lit. three-necked flask were introduced 305 g (1 mole) of 2-amino-ethanol-bis-(4-methyl-3,4-epoxy-cyclohexenecarboxylate), and further thereto, 770 g of an acetone solution having a peracetic acid content of 25% by weight [containing 192 g (2.5 mol) of peracetic acid] was dropwise added over a period of 4 hrs., while maintaining the internal temperature at 35 to 40 °C. After completion of addition, the reaction continued further for 4 hrs. at the same temperature. The reaction mixture was allowed to stand for one night at -11 °C and then, the residual amount of peracetic acid was checked and it was confirmed that at least 98% of the theoretical amount was reacted.

Subsequently, the reaction mixture was diluted with 1 lit. of toluene and heated at 50 °C under reduced pressure using a water-jet aspirator to distil away low boiling components until no distillate was formed. The remained reaction composition was subjected to vacuum distillation to obtain an objective compound. The yield was 77%.

### Synthetic example 5

### Synthesis of Exemplified compound EP-41 (3-Amino-2,2-dimethyl-1-propane-1-ol-bis-(4-methyl-3,4-epoxycyclohexanecarboxylate)

### [Synthesis of (3-Amino-2,2-dimethyl-1-propane-1-ol-(4-methyl-3-cyclohexenecarboxylate))

To a mixture of 308 g (2 moles) of methyl-(4-methyl-3-cyclohexenecarboxylate) and 103 g (1 mole) of 3-amino-2,2-dimethyl-propane-1-ol was added 1 g of phosphoric acid, and the resulting mixture was reacted at 110 to 120 °C for 12 hrs. The reaction mixture was washed with an aqueous sodium bicarbonate solution and subjected to vacuum distillation to obtain an objective compound. The yield was 86%.

Into 2 lit. three-necked flask were introduced 347 g (1 mole) of 3-amino-2,2-dimethyl-1-propane-1-ol-(4-methyl-3-cyclohexenecarboxylate and further thereto, 770 g of an acetone solution having a peracetic acid content of 25% by weight [containing 192 g (2.5 mol) of peracetic acid] was dropwise added over a period of 4 hrs., while maintaining the internal temperature at 35 to 40 °C. After completion of addition, the reaction continued further for 4 hrs. at the same temperature. The reaction mixture was allowed to stand for one night at -11 °C and then, the residual amount of peracetic acid was checked and it was confirmed that at least 98% of the theoretical amount was reacted.

Subsequently, the reaction mixture was diluted with 1 lit. of toluene and heated at 50 °C under reduced pressure using a water-jet aspirator to distil away low boiling components until no distillate was formed. The remained reaction composition was subjected to vacuum distillation to obtain an objective compound. The yield was 75%.

Other epoxy compounds of the invention can be synthesized in the same manner as above at good yield.

The content of the epoxy compound represented by formula (1) or (2) above in the composition or actinic energy radiation hardenable composition of the invention is preferably from 10 to 70% by weight, and more preferably from 20 to 50% by weight.

The epoxy compound in the invention has a molecular weight of preferably not less than 170, in view of safety. from 170 to 1,000, and more preferably from 300 to 700. When the epoxy compound is used in ink jet ink, the molecular weight of the epoxy compound is not more than 1000, in view of its viscosity. It is more preferred in the epoxy compound that the value obtained by dividing the molecular with the epoxy group number in the molecule weight is from 160 to 300.

### (Oxetane compound)

The oxetane compound used in the invention is a compound having one or more oxetane rings in the molecule. Typical examples of the oxetane compound include 3-ethyl-3-hydroxymethyloxetane (OXT101, etc.), 1,4-bis [(3-ethyl-3-oxetanyl)-methoxymethyl]benzene (OXT 121 etc.), 3-ethyl-3-(phenoxymethyl)oxetane (OXT 211 etc.), di(1-ethyl-3-oxetanyl) methyl ether (OXT 221 etc.), and .), and 3-ethyl-3-(2-ethylhexyloxymethyl)oxetane (OXT 212 etc.), each produced by Toa Gosei Co., Ltd. Especially preferred are 3-ethyl-3-hydroxymethyloxetane, 3-ethyl-3-(phenoxymethyl)oxetane and di(1-ethyl-3-oxetanyl) methyl ether. These can be used singly or as a mixture of two or more thereof.

The content of the oxetane compound in the actinic energy radiation hardenable composition of the invention is preferably from 30 to 95% by weight, and more preferably from 50 to 80% by weight.

In the actinic energy radiation hardenable composition of the invention, an oxirane ring-containing compound other than the epoxy compound of the invention can be used in combination. The oxirane ring-containing compound is a compound containing in the molecule one or more oxirane rings represented by the following formula:

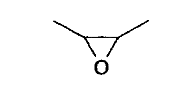

Generally, a monomer, oligomer or polymer each having an oxirane ring can be used as an epoxy resin. Examples thereof include known aromatic epoxides, alicyclic epoxides, and aliphatic epoxides. Hereinafter, "epoxide" implies a monomer or an oligomer. These compounds may be used singly or in combination.

A preferable aromatic epoxide is a di- or poly-glycidyl ether manufactured by a reaction of polyhydric phenol having at least one aromatic ring or of an alkylene oxide adduct thereof with epichlorohydrin, and includes, for example, such as di- or poly-glycidyl ether of bisphenol A or of an alkylene oxide adduct thereof, di- or poly-glycidyl ether of hydrogenated bisphenol A or of an alkylene oxide adduct thereof and novolac type epoxy resin. Herein, alkylene oxide includes such as ethylene oxide and propylene oxide.

An alicyclic epoxide is preferably a compound containing cyclohexene oxide or cyclopentene oxide obtained by epoxydizing a compound having at least one cycloalkane ring such as cyclohexene or cyclopentene by use of a suitable oxidizing agent such as hydrogen peroxide or a peracid. Examples of the alicyclic epoxide include celloxide 2021, celloxide 2021A, celloxide 2021P, celloxide 2080, celloxide 2000, Epolead GT301, Epolead GT302, Epolead GT401, Epolead GT403, EHPE-3150, EHPEL-3150, each produced by Daicel Kagaku Kogyo Co., Ltd.; and UVR-6105, UVR-6110, UVR-6128, UVR-6100, UVR-6216, UVR-6000, each produced by Union Carbide Co., Ltd.

A preferable aliphatic epoxide is such as di- or polyglycidyl ether of aliphatic polyhydric alcohol or of an alkylene oxide adduct thereof; the typical examples include diglycidyl ether of alkylene glycol, such as diglycidyl ether of ethylene glycol, diglycidyl ether of propylene glycol and diglycidyl ether of 1,6-hexane diol; polyglycidyl ether of polyhydric alcohol such as di- or triglycidyl ether of glycerin or of an alkylene oxide adduct thereof; and diglycidyl ether of polyalkylene glycol such as diglycidyl ether of polyethylene glycol or of an alkylene oxide adduct thereof and diglycidyl ether of polypropylene glycol or of an alkylene oxide adduct thereof. Herein, alkylene oxide includes such as ethylene oxide and propylene oxide.

Besides the compounds described above, monogycidyl ethers of higher aliphatic alcohols, phenol or cresol can be used. Among these epoxides, the aromatic epoxide and alicyclic epoxide are preferable and the alicyclic epoxide is specifically preferable, taking a quick curing property in consideration.

These oxirane ring-containing compounds are contained in an amount of 0 to 50% by weight, and preferably from 0 to 30% by weight in the actinic energy radiation hardenable composition of the invention.

### (Vinyl ether compound)

Examples of the vinyl ether compound contained in the actinic energy radiation hardenable composition of the invention include di- or tri-vinyl ether compounds such as ethylene glycol divinyl ether, ethylene glycol monovinyl ether, diethylene glycol divinyl ether, triethylene glycol divinyl ether, propylene glycol divinyl ether, dipropylene glycol divinyl ether, butane diol divinyl ether, hexane diol divinyl ether, cyclohexane dimethanol divinyl ether, and trimethylol propane trivinyl ether; and mono vinyl ether compounds such as ethyl vinyl ether, n-butyl vinyl ether, iso-butyl vinyl ether, octadecyl vinyl ether, cyclohexyl vinyl ether, hydroxybutyl vinyl ether, 2-ethylhexyl vinyl ether, cyclohexane dimethanol monovinyl ether, n-propyl vinyl ether, isopropyl vinyl ether, isopropenyl ether-o-propylene carbonate, dodecyl vinyl ether, diethylene glycol monovinyl ether, and octadecyl vinyl ether.

In these vinyl ether compounds, when the hardenability, adhesion or surface hardness is considered, di- or tri-vinyl ether compounds are preferable, and particularly divinyl ether compounds are preferable. In the present invention, these vinyl ether compounds may be used alone or as an admixture of two or more kinds thereof.

Addition of the vinyl ether compound to the actinic energy radiation hardenable composition of the invention can adjust viscosity of the composition, and can increase the hardening rate of the composition. The content of the vinyl ether compound in the actinic energy radiation hardenable composition of the invention is preferably from 0 to 40% by weight, and more preferably from 0 to 20% by weight.

### (Cationic photopolymerization initiator)

Examples of the cationic photopolymerization initiator used in the invention include arylsulfonium derivatives (for example, Silacure UVI-6990 or Silacure UVI-6974 produced by Union Carbide Co., Ltd., or Adekaoptomer SP-150, Adekaoptomer SP-152, Adekaoptomer SP-170, or Adekaoptomer SP-172 produced by Asahi Denka Kogyo Co., Ltd.); aryliodonium derivatives (for example, RP-2074 produced by Rodia Co., Ltd.); Arene-ion complexes (for example, Irgacure 261 produced by Ciba Geigy Co., Ltd.); diazonium salts; triazine type initiator; and other halogenides.

The cationic photopolymerization initiator content is preferably from 0.2 to 20 parts by weight based on 100 parts by weight of a compound having an alicyclic epoxide group. The content less than 0.2 parts by weight provides a poor hardening property, and the content exceeding 20 parts by weight not exhibit a further hardening property. These cationic photopolymerization initiators may be used singly or as a mixture of two or more kinds thereof.

In the invention, a sulfonium salt represented by one of formula (4) through (7), releasing no benzene on exposure of an actinic energy radiation, is suitably used.

Herein, "a sulfonium salt releasing no benzene on exposure of an actinic energy radiation" refers to a sulfonium salt which does not substantially release benzene on exposure of an actinic ray, and particularly a sulfonium salt such that when a 15 µm thick image with an area of 100 m² Is formed employing ink containing the sulfonium salt in an amount of 5% by weight, and the resulting image is sufficiently exposed at 30 °C to actinic rays so as to completely decompose the sulfonium salt, a releasing amount of benzene is not more than 5 µg or zero.

The sulfonium salt is preferably a sulfonium salt represented by one of formula (4) through (7), and the sulfonium salt, which has a substituent on the benzene ring bonding the ⁺S satisfies the above mentioned definition.

In formulae (4) through (7) above, R₁ through R₁₇ independently represent a hydrogen atom or a substituent, provided that R₁ through R₃ are not simultaneously hydrogen atoms, R₄ through R₇ are not simultaneously hydrogen atoms, R₈ through R₁₁ are not simultaneously hydrogen atoms, and R₁₂ through R₁₇ are not simultaneously hydrogen atoms.

Examples of the substituent represented by R₁ through R₁₇ include an alkyl group such as a methyl group, an ethyl group, a propyl group, an isopropyl group, a butyl group, an isobutyl group, a t-butyl group, a pentyl group, or a hexyl group; an alkoxy group such as a methoxy group, an ethoxy group, a propoxy group, an isopropyl group, a butoxy group, a hexyloxy group, a decyloxy group or a dodecyloxy group; a carbonyl group such as an acetoxy group, a propionyloxy group, a decylcarbonyloxy group, a dodecylcarbonyloxy group, a methoxycarbonyl group, an ethoxycarbonyl group or a benzoyloxy group; a phenylthio group; a halogen atom such as fluorine, chlorine, bromine or iodine; a cyano group; a nitro group; and a hydroxyl group.

X represents a non-nucleophilic anion. Examples thereof include a halogen ion such as F, Cl, Br or I, B(C₆F₅)₄, R₁₈COO, R₁₉SO₃, SbF₆, AsF₆, PF₆, and BF₄, in which R₁₈ and R₁₉ independently represent an alkyl group such as a methyl group, an ethyl group, a propyl group or a butyl group; an alkyl group having, as a substituent, a halogen atom such as fluorine, chlorine, bromine or iodine, a nitro group, a cyano group, a methoxy group or an ethoxy group; or a phenyl group. Among these, B(C₆F₅)₄ and PF₆ are preferred in view of safety.

The above compounds can be easily synthesized according to commonly known methods, for example, in the same manner as the photolytically acid generating agent described in "THE CHEMICAL SOCIETY OF JAPAN", Vol. 71, No. 11 (1998), and "Imejinguyou Yukizairyo", edited by Yuki Erekutoronikus Zairyokenkyukai, and published by Bunshin Shuppan (1993).

In the invention, the sulfonium salt represented by one of formulae (4) through (7) is preferably at least one selected from the sulfonium salts represented by formulae (8) through (16). X represents a non-nucleophilic anion as described above.

Examples of a photopolymerization promoting agent include anthracene; and anthracene derivatives (for example, Adekaoptomer SP-100 produced by Asahi Denka Kogyo Co., Ltd.). These photopolymerization promoting agents may be used singly or as a mixture of two or more kinds thereof.

### (Pigment)

As the pigments contained in the actinic energy radiation hardenable composition of the invention, there are various pigments such as organic or inorganic pigments. Examples of the pigment include white pigments such as titanium oxide, zinc white, white lead, lithopone and antimony oxide; black pigments such as aniline black, iron black and carbon black; yellow pigments such as chrome yellow, yellow oxide, hansa yellow (100, 50, 30 etc.), titan yellow, benzine yellow and permanent yellow; orange pigments such as chrome vermilion, permanent orange, Vulcan fast orange, and indanthrene brilliant orange; brown pigment such as iron oxide, permanent brown and parabrown; red pigments such as red iron oxide, cadmium red, antimony vermilion, permanent red, rhodamine lake, alizarin lake, thioindigo Red, PV carmine, monolite fast red, and quinacridone; violet pigments such as cobalt violet, manganese violet, fast violet, methyl violet lake, indanthrene brilliant violet, and dioxazine violet; blue pigments such as ultramarine blue, Prussian blue, cobalt blue, alkali blue lake, metal free phthalocyanine blue, copper phthalocyanine blue, indanthrene blue, and indigo; green pigments such as chrome green, zinc green, chromium oxide, emerald green, naphthol green, green gold, acid green lake, malachite green lake, phthalocyanine green, polychlorobromo copper phthalocyanine, and various kinds of fluorescent pigment, metal powder pigment and extender pigment.

The content of the pigments in the actinic energy radiation hardenable composition of the invention is preferably from 3 to 50% by weight, and more preferably from 5 to 20% by weight, in obtaining sufficient image density or sufficient light fastness.

The actinic energy radiation hardenable composition of the invention optionally contains the following components in an amount of not more than 5% by weight.

As the components used in printing ink, a coating for cans, plastics, paper or wood materials, an adhesive or ink-jet ink, thcrc arc inorganic fillers, a softening agent, an anti-oxidant, an anti-aging agent, a stabilizer, an adhesion-providing agent, a leveling agent, an anti-foaming agent, a plasticizer, a dyestuff, a processing agent, an organic solvent, a lubricant, and an ultraviolet light-shielding agent. Examples of the polymeric binder include polyesters, polyurethanes, vinyl resins, acryl resins, rubber resins, and waxes. Examples of the inorganic fillers include metal oxides such as zinc oxide, aluminum oxide, antimony oxide, calcium oxide, chromium oxide, tin oxide, titanium oxide, iron oxide, copper oxide, lead oxide, bismuth oxide, magnesium oxide and manganese oxide; hydroxides such as aluminum hydroxide, ferrous hydroxide, and calcium hydroxide; salts such as calcium carbonate and calcium sulfate; a silicon compound such as silicon dioxide; natural pigment such as kaolin, bentonite, clay and talc; minerals such as natural zeolite, ohya stone, natural mica and ammonite; synthetic inorganic substances such as artificial mica and artificial zeolite; and metals such as aluminum, iron and zinc. In these components, there are those overlapping with the pigments described above, however, these can be added to the composition or ink of the invention as additional additives. The lubricant is added in order to increase lubricity of a coating surface, and examples thereof include waxes such as fatty acid ester wax which is ester of polyol and fatty acid, silicon wax, fluorine wax, polyolefin wax, animal wax, and vegetable wax. Examples of the adhesion-providing agent include rosins such as resin acid, polymeric resin acid, and resin acid ester; terpene resin; terpene-phenol resin; aromatic hydrocarbon resin; aliphatic saturated hydrocarbon resin; and petroleum resin.

The actinic energy radiation hardenable composition of the invention used in an optical solid shaping system can further contain a thermoplastic polymer. The thermoplastic polymer is liquid or solid at room temperature, and one uniformly miscible with other components in the composition at room temperature. Typical examples of the thermoplastic polymer include polyesters, polyvinyl acetate, polyvinyl chloride, polybutadiene, polycarbonate, polystyrene, polyvinyl ether, polyvinyl butyral, polyacrylates, polymethyl methacrylate, polybutene and a hydrogenated styrene-butadiene block copolymer. There can be used those in which a hydroxyl group, a carboxyl group a vinyl group or an epoxy group is incorporated into these thermoplastic polymers. The number average molecular weight of these thermoplastic polymers is preferably from 1000 to 500000, and more preferably from 5000 to 100000. Those having a molecular weight falling outside the range may be used. However, those having too low molecular weight are not sufficient to improve strength, while those having too high molecular weight increase the viscosity of a composition containing those, and are undesirable as resin used for an optical solid shaping resin composition.

A method, which the components described above are mixed to obtain the actinic energy radiation hardenable composition of the invention, is not specifically limited as long as the components are sufficiently mixed. As the mixing method, there are a stirring method employing rotation of a propeller, a kneading method employing kneading rollers, and a dispersion method employing a common disperser such as a sand mill.

As actinic ray used for hardening the actinic energy radiation hardenable composition of the invention, there are ultraviolet ray, electron beam, X-ray, radioactive ray, and high frequency wave. Among these, ultraviolet ray is most preferred in cost saving. As an ultraviolet ray source, there are ultraviolet laser, a mercury lamp, a xenon lamp, a sodium lamp, and an alkali metal lamp. When concentration of rays is necessary, laser rays are especially preferred.

Utilizing methods of the actinic energy radiation hardenable composition will be described below based on its applications.

In the case of printing ink application, the actinic energy radiation hardenable composition can be utilized in various types of printing methods such as planographic printing such as off set printing, letter press printing, silk screen printing or gravure printing employing a substrate such as paper, film or a sheet. The composition is cured by actinic energy radiation irradiation after printing. Examples of the actinic energy radiation include ultraviolet rays, X rays and electron rays. As a light source used in ultraviolet ray irradiation, various types of light sources can be used. Examples thereof include an increased pressure or high pressure mercury lamp, a metal halide lamp, a xenon lamp, a non-electrode lamp and a carbon arc lamp. When hardening is carried out employing electron rays, various types of irradiation devices can be employed which include irradiation devices of Cockcroft-Walton's type, Van de Graaff's type or a resonance transformer type. The electron rays have energy of preferably from 50 to 1,000 eV and more preferably from 100 to 300 eV. In the invention, it is preferable to utilize ultraviolet rays to harden the actinic energy radiation hardenable composition since an inexpensive device can be used.

In application to coatings for cans, plastics, papers or wood materials, the composition of the invention is applied to coatings for metal materials, plastic materials, papers or wood materials. Examples of the metal materials include an electroplated steel plate, a tin-free steel, and aluminum. Examples of the plastic materials include polycarbonate, polymethyl methacrylate, polyethylene terephthalate, polyvinyl chloride, and ABS resin. Examples of the papers include plain paper comprised mainly of cellulose and paper which is laminated with polyethylene, polyvinyl chloride, polypropylene, polyester, polycarbonate or polyimide. Examples of the wood materials include natural trees such as a cherry tree, a red oak, a rosewood, a Chinese quince, mahogany, lauan, a mulberry tree, a box tree, a Japanese nutmeg, a Chinese cork tree, a honoki, a katsura tree, a keyaki, a walnut, a camphor tree, a Japanese oak, a teak, a persimmon tree, a Jindai katsura tree, a Jindai sugi, a black persimmon tree, an ebony, a tochinoki, a maple tree, a willow and an ash tree; artificial tree materials such as plywood, laminated boards, particle boards and printed plywood, and floors, furniture or walls comprised of these natural or artificial trees. These materials may be in the form of plate or sheet. The coating thickness of the composition of the invention coated on a substrate is optional according to its application, and the preferred thickness is preferably from 1 to 50 µm, and more preferably from 3 to 20 µm.

The coating method of the composition of the invention is not specifically limited, and may be carried out employing a conventional coating method, for example, a dipping method, a flow coating method, a spray coating method, a bar coating method, a gravure coating method, a roll coating method, a blade coating method, or an air-knife coating method. The composition of the invention is coated on a substrate employing a coating apparatus to form a coating layer, and irradiated with actinic energy radiation to harden the coating layer. Examples of the actinic energy radiations include ultraviolet rays, X rays and electron rays. As a light source used in ultraviolet ray irradiation, various types of light sources can be used. Examples thereof include an increased pressure or high pressure mercury lamp, a metal halide lamp, a xenon lamp, a non-electrode lamp and a carbon arc lamp. When hardening is carried out employing electron rays, various types of irradiation devices can be employed which include irradiation devices of Cockcroft-walton's type, Van de Graaff's type or a resonance transformer type. The electron rays have energy of preferably from 50 to 1,000 eV and more preferably from 100 to 300 eV. In the invention, it is preferable to utilize ultraviolet rays to harden the actinic energy radiation hardenable composition since an inexpensive device can be used.

After the composition of the invention is coated on the plastic materials, the resulting materials are optionally subjected to processing such as molding, printing or transferring. In molding, after a substrate coated with the composition of the invention is heated to appropriate temperature, the substrate is subjected to processing such as vacuum molding, vacuum and pressure molding, pressure molding or mat molding, or only the coated layer on the substrate is subjected to embossing to give on the surface a convexoconcave such as an interference band in the same manner as copying of CD or records. In printing, printing is carried out employing a conventional printing press to print on then coated layer. In transferring, the composition of the invention is coated on a substrate such as a polyethylene terephthalate film, optionally subjected to printing or embossing as described above, coated with an adhesive, and transferred to another substrate.

In application to adhesives, the composition of the invention is applied without any limitations, and may be applied in accordance with a conventional method as carried out in manufacturing laminates. For example, the composition of the invention is coated on a first thin-layer coating substrate, optionally dried, then laminated with a second thin-layer coating substrate, and the resulting laminate is subjected to an actinic energy radiation exposure. Herein, at least one of the thin-layer coating substrates should be a plastic film. Examples of the thin layer substrate include plastic films, paper sheets and metal foils. Herein, the plastic films refer to ones capable of transmitting an actinic energy radiation. The thickness thereof is determined according to usage or kinds thereof, but is preferably not more than 0.2 mm. Examples of the plastic films include those of polyvinyl chloride, polyvinylidene chloride, celluloses, polyethylene, polypropylene, polystyrene, ABS resin, polyamide, polyester, polyurethane, polyvinyl alcohol, ethylene-vinyl acetate copolymer and chlorinated polypropylene. Examples of the papers include simili paper, woodfree paper, kraft paper, art coat paper, caster coat paper, white roll paper, parchment paper, waterproof paper, glassine paper, paper for corrugated board. As the metal foils, there is, for example, an aluminum foil. Coating on a thin substituent can be carried out according to a conventional method such as a method employing a natural coater, a knife belt coater, a floating knife coater, a knife over-roll coater, a knife on blanket coater, a spray coater. a dipping coater, a kiss roll coater, a squeeze roll coater, a reverse roll coater, an air-blade coater, a curtain flow coater or a gravure coater. The coating thickness of the composition of the invention is determined according to usage or kinds of substrates on which the composition is coated, but is preferably from 0.1 to 1000 *µ*m, and more preferably from 1 to 50 *µ*m. Examples of the actinic energy radiation include ultraviolet rays, X rays and electron rays. As a light source used in ultraviolet ray irradiation, various types of light sources can be used. Examples thereof include an increased pressure or high pressure mercury lamp, a metal halide lamp, a xenon lamp, a non-electrode lamp and a carbon arc lamp. When hardening is carried out employing electron rays, known irradiation devices can be used as EB irradiation devices, and various types of irradiation devices can be employed. Examples thereof include irradiation devices of Cockcroft-Walton's type, Van de Graaff's type or a resonance transformer type. The electron rays have energy of preferably from 50 to 1,000 eV and more preferably from 100 to 300 eV. In the invention, it is preferable to utilize ultraviolet rays to harden the actinic energy radiation hardenable composition since an inexpensive device can be used.

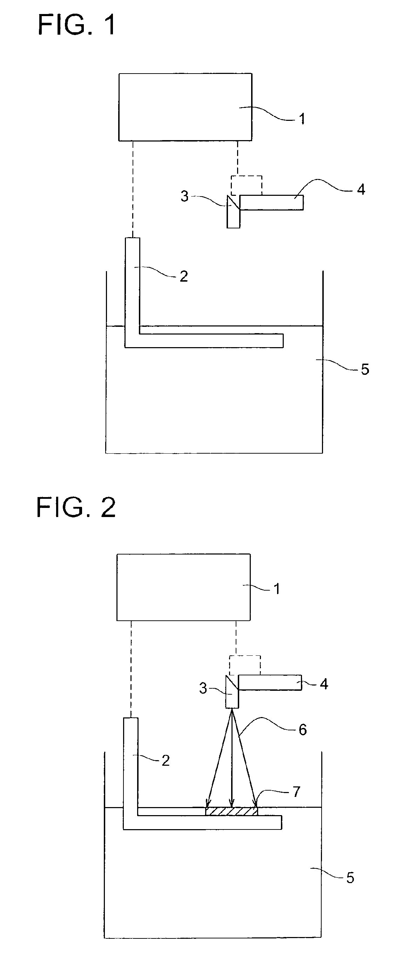

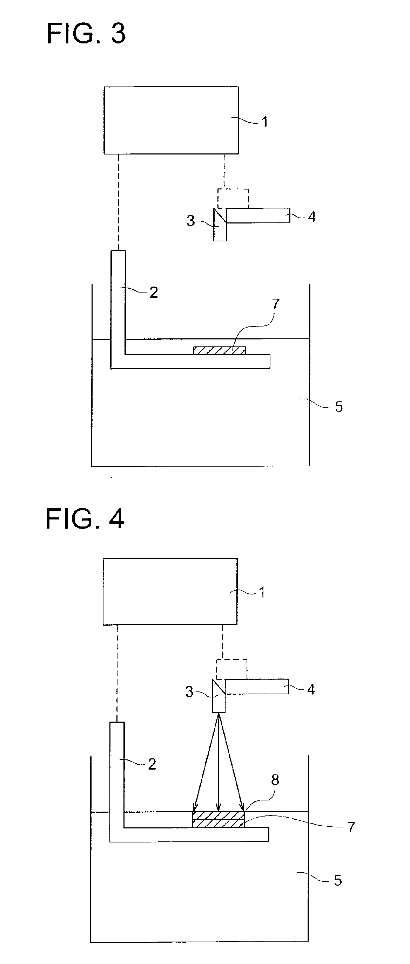

In the use in optical three-dimensional shaping system, a first unhardened layer comprised of the actinic energy radiation hardenable composition of the invention is exposed to energy radiation, hardened at exposed portions to form a first hardened layer with intended thickness, supplied with the actinic energy radiation hardenable composition on the first hardened layer to form a second unhardened layer, and further exposed to energy radiation, whereby a second hardened layer is formed on the first hardened layer. When this process is repeated, a three-dimensional object is obtained. This process will be explained in detail below, employing drawings. In Fig. 1, NC table 2 is placed in a composition 5 to form on table 2 a first unhardened layer with a depth corresponding to intended pitch. Subsequently, the first unhardened layer is scanning exposed to laser beams 6 from a laser 4 by controlling optical system 3 according to signals from control section 1 based on CAD data, whereby a first hardened layer 7 is formed (Fig. 2). Subsequently, NC table 2 is further sunken in the composition by signals from control section 1 to form on the first hardened layer 7 a second unhardened layer with a depth corresponding to intended pitch (Fig. 3). The second unhardened layer is scanning exposed to laser beams 6 to obtain a second hardened layer 8 (Fig. 4). This process is repeated to obtain multilayers.

The ink-jet ink composition is manufactured by appropriately dispersing pigment in addition to an ultraviolet ray curable compound and a pigment dispersant via an ordinary homogenizer such as a sand mill. It is preferable to prepare in advance a highly concentrated solution of pigment and which is diluted with an active ray hardenable compound. Since sufficient dispersion is possible with ordinary homogenizers, negating excess dispersion energy, nor is much dispersion time required, whereby barely modifying ink components at the time of dispersion, resulting in preparation of ink exhibiting excellent overall stability. Ink is preferably filtered employing a filter of less than 3 µm, more preferably less than 1 µm.

The average particle size of the pigment is preferably from 0.08 to 0.5 µm. The dispersion condition, filtration condition or kinds of the pigment, pigment dispersant or dispersion medium are suitably selected to obtain pigment having a maximum particle size falling in the range of from 0.3 to 10 µm, and preferably from 0.3 to 3 µm. This particle size range prevents clogging of ink-jet head nozzle, and maintains storage stability, transparency or hardening sensitivity of the ink.

The pigment content of the ink-jet ink composition is preferably from 1 to 10% by weight.

Viscosity of the ink-jet ink composition is adjusted to be higher, and preferably from 5 to 50 mPa·s at 25 °C. Ink having a viscosity of 5 to 50 mPa·s at 25 °C exhibits stable ejection characteristics, even employing a head with a frequency of as high as 10 to 50 kHz as well as a head with a common frequency of 4 to 10 kHz. When the viscosity is less than 5 mPa·s, deterioration of ejection following property at high frequency is observed, while when the viscosity is over 50 mPa·s, ejection property deteriorates even with a viscosity lowering mechanism such as heating the head, resulting in poor ejection stability and ejection impossibility.

Further, the ink-jet ink composition is preferably one which has a conductivity of not higher than 10 µs/cm at a piezo head and does not cause electrical corrosion at the interior of the head. Further, in a continuous type, the conductivity is necessarily adjusted by electrolyte, and in this case, the conductivity should be adjusted to not lower than 0.5 mS/cm.

In this invention, surface tension of ink at 25 °C is preferably in a range of 25 - 40 mN/m. In the case of surface tension of ink being less than 25 mN/m, stable ejection is hardly realized, while in the case of over 40 mN/m, an aimed dot diameter can not be achieved. Out of a range of 25 - 40 mN/m, it becomes difficult to obtain a uniform dot diameter against various supports even with light irradiation while controlling viscosity and water content of ink as described in the invention.

A surfactant may be appropriately incorporated to adjust the surface tension. The surfactants used in the invention include anionic surfactants such as dialkylsulfosuccinates, alkylnaphthalenesulfonates and fatty acid salts; nonionic surfactants such as polyoxyethylene alkyl ethers, polyoxyethylene alkylallyl ethers, acetylene glycols and polyoxyethylene-polyoxypropylene block copolymers; cationic surfactants such as alkylamines and quaternary ammonium salts; and surface active compounds having a polymerizing group. Among them, specifically preferred are surface active compounds having a polymerizing group such as an unsaturated bond and an oxirane or oxetane ring, such as silicone modified acrylate, fluorine modified acrylate, silicone modified epoxy, fluorine modified epoxy, silicone modified oxetane and fluorine modified oxetane.

In the ink-jet ink composition, various additives other than those explained above can be utilized. For example, added can be a leveling agent, a matting agent; polyester type resin, polyurethane type resin, vinyl type resin, acrylic type resin, rubber type resin and waxes to adjust film physical properties. Addition of a slight amount of an organic solvent is also effective to improve adhesion with a recording medium. In this case, addition in a range of not causing problems of solvent resistance and VOC is effective, and the using amount is in a range of 0.1 - 5% and preferably of 0.1 - 3%. Further, by combining with radical polymerizing monomer and an initiator, it is also possible to make radical·cation hybrid type hardenable ink.

In the image forming method of the invention, the ink jet ink composition in the invention is ejected onto a recording material by an ink-jet recording head containing at least one nozzle and successively the ejected ink composition is hardened by irradiation of actinic rays such as ultraviolet rays.

In the image forming method of the invention, the ink-jet ink composition in the invention is ejected onto a recording material by an ink-jet recording head containing at least one nozzle and successively the ejected ink composition is hardened by irradiation of actinic rays such as ultraviolet rays.

In the image forming method of the invention, it is preferable to heat ink together with the ink-jet nozzle to decrease the ink viscosity. The heating temperature is commonly 30 to 80 °C, and preferably 35 to 60 °C.

In the invention, the thickness of an ink layer, after ink has been ejected onto a recording medium and hardened by ultraviolet ray irradiation, is preferably from 2 to 20 µm. In ink jet recording employing ultraviolet ray curable ink, the total thickness of the ink on the recording medium is at present over 20 µm in the screen printing field. Excessive ink cannot be ejected onto the recording medium in the flexible package printing field where a thin plastic film is used as recording medium, because problems are caused in that stiffness and texture of printed matter vary, in addition to problems of the aforementioned curl and wrinkles of the recording medium. In the invention, a volume of the photocurable ink droplets ejected from nozzles is preferably 2 to 15 p1.

In the invention, it is preferred that the irradiation timing is as early as possible in order to form an image with high resolution. The ultraviolet ray irradiation is preferably started at timing when the ink viscosity or moisture content is in a preferable state.

It is preferred that ultraviolet ray is irradiated 0.001 to 2.0 seconds after ink has been ejected on recording medium, and it is more preferred that actinic ray is irradiated 0.001 to 0.4 second after ink has been ejected on recording medium. It is preferred that the irradiation has been carried out until ink fluidity is lost, and is completed in 0.1 to 3 seconds, preferably in 0.2 to 1 seconds. This can prevent undesired enlargement of dots or blurring of dots.

As an ultraviolet ray irradiation method, a basic method is disclosed in JP-A No. 60-132767, in which light sources are provided at the both sides of a head unit where a head and a light are scanned in a shuttle mode. Irradiation is performed in a certain time interval after ink has been ejected onto recording medium. Further, curing is completed by another light source which is not driven. As a light irradiation method, a method utilizing optical fiber, and a method in which collimated light source is reflected by a mirror provided on the side surface of a head unit and UV light (ultraviolet light) is irradiated on a recording portion are disclosed in USP No. 6145979. In an image forming method of the invention, any of these irradiation methods can be utilized.

Further, a preferable embodiment is a method in which irradiation of ultraviolet rays is divided into two steps to firstly irradiate ultraviolet rays according to the aforesaid method within 0.001 to 2.0 seconds after ink landing, and ultraviolet rays are further irradiated after finishing the whole print. By dividing irradiation of the ultraviolet rays into two steps, it is possible to restrain shrinkage of a recording material which is caused during ink hardening.

Examples of a light source utilized for ultraviolet ray irradiation include a mercury arc lamp, a xenon arc lamp, a fluorescent lamp, a carbon arc lamp, a tungsten-halogen copying lamp, a high pressure mercury lamp, a metal halide lamp, a non-electrode UV lamp, a low pressure mercury lamp, a UV laser, a xenon flush lamp, an insect catching lamp, a black light, a sterilizing lamp, a cold cathode tube and a LED, however, the light source is not limited thereto. Among them preferable is a fluorescent lamp because of low energy and low cost. Wavelength of the light source is preferably one having an emission peak in the range of from 250 to 370 nm, and more preferably 270 to 320 nm, in view of sensitivity. The illuminance is from 1 to 3,000 mW/cm² and preferably from 1 to 200 mW/cm². When electron rays are employed for hardening, hardening is ordinarily carried out employing electron rays having energy of not higher than 300 eV, however, instantaneous hardening can be also carried out with an irradiation amount of 1 to 5 Mrad.

A printing image is recorded on an ink-jet recording medium (hereinafter also referred to as a substrate) employing the ink-jet ink composition in the invention. As materials for the ink-jet recording medium, conventional synthetic resins widely used for various use can be used. Examples of the resins include polyester, polyethylene, polyurethane, polypropylene, acryl resin, polycarbonate, polystyrene, acrylonitrile-butadiene styrene copolymer, polyethylene terephthalate, and polybutadiene terephthalate. Thickness or form of these resins is not specifically limited.

As the recording medium used in the invention, ordinary non-coated paper or coated paper, or non-absorptive recording sheets can be utilized. Among them, non-absorptive recording sheets are preferred.

As the non-absorptive recording sheets used in the invention, various non-absorptive plastic films can be used. Examples of the plastic films include, for example, a PET (polyethylene terephthalate) film, an OPS (oriented polystyrene) film, an OPP (oriented polypropylene) film, an ONy (oriented nylon) film, a PVC (polyvinyl chloride) film, a PE (polyethylene) film and a TAC (triacetylcellulose) film. Plastic films other than these, polycarbonate, acryl resin, ABS, polyacetal, PVA and a rubber series can be utilized. A metal series and a glass series are also applicable. The invention is effective especially in forming an image on a PET film, an OPS film, an OPP film, an ONy film or a PVC film, which are capable of thermal shrinking, among the above recording films. These films are liable to cause curl and deformation of film due to such as curing shrinkage of ink or heat accompanied with curing reaction of ink, and, in addition, the formed ink layer is hard to follow shrinkage of the films.

Plastic films greatly differ in surface energy depending on the kinds, and heretofore, there has been a problem in that the ink dot diameter after ink deposition on recording medium varies depending on the kinds of the recording mediums. The recording mediums used in the invention ranges from an OPP or OPS film each having a low surface energy to a PET film having a relatively high surface energy. In the invention, the recording mediums have a surface energy of preferably from 40 to 60 mN/m.

In the invention, a long length web recording medium is advantageously used in view of recording medium cost such as production cost and packaging cost, image recording efficiency, or adaptability to various sizes of prints.

### EXAMPLES

The invention will be explained blow employing examples, however, the invention is not limited thereto.

### (Preparation of actinic energy radiation hardenable composition Nos. 1 through 9)

A mixture of the components other than the cationic photopolymerization initiator (parts by weight) was dispersed in a sand mill for 4 hours to obtain an actinic energy radiation hardenable composition liquid. Thereafter, a cationic photopolymerization initiator as shown in Table 1 was added in an amount as shown in Table 1 to the resulting liquid, mildly mixed, and filtered under pressure employing a membrane filter to obtain an actinic energy radiation hardenable composition. Thus, inventive actinic energy radiation hardenable composition Nos. 1 through 9 were obtained.

**Table 1**

| Actinic energy radiation hardenable composition No . | Pigment used (^{*1} Content) | Epoxy compound used (^{*1}Content) | Oxetane compound used (^{*1}Content) | Epoxy compound used (^{*1}Content) | Vinyl ether compound used (^{*1}Content) | Cationic photopolymerization initiator used (^{*1}Content) | Remarks |
|---|---|---|---|---|---|---|---|
| 1 | P1 (5) | EP11 (15) | ^{*2}a (65) | None ^{*4}C | (10) | SP-1 (5) | Inv. |
| 2 | P1 (5) | EP15 (20) | ^{*2}a (70) | None | None | SP-1 (5) | Inv. |
| 3 | P1 (5) | EP21 (20) | ^{*2}a (70) | None | None | SP-2 (5) | Inv. |
| 4 | P1 (5) | EP31 (20) | ^{*2}a (70) | None | None | SP-2 (5) | Inv. |
| 5 | None | EP41 (10) | ^{*2}a (70) | ^{*3}b (10) | None | SP-3 (10) | Inv. |
| 6 | P1 (5) | EP-A (15) | ^{*2}a (65) | None | ^{*4}C (10) | SP-1 (5) | Comp. |
| 7 | P1 (5) | EP-B (20) | ^{*2}a (70) | None | None | SP-1 (5) | Comp. |
| 8 | P1 (5) | EP-C (20) | ^{*2}a (70) | None | None | SP-2 (5) | Comp. |
| 9 | None | EP-C (10) | ^{*2}a (70) | ^{*3}b (10) | None | SP-3 (10) | Comp. |

| | | | | | | | |
|---|---|---|---|---|---|---|---|
| Inv.: Inventive, Comp.: Comparative *1: Content is represented by parts by weight. *2: a represents OXT221. *3: b represents Celloxide 3000. *4: c represents DVE-3. | | | | | | | |

In Table 1, "content" is represented in terms of parts by weight. Details of the components in Table 1 are as follows:

### Pigment

P1: Crude copper phthalocyanine ("Copper phthalocyanine" produced by Toyo Ink Manufacturing Co., Ltd.) of 250 parts, 2500 parts of sodium chloride, and 160 parts of polyethylene glycol (Polyethylene glycol 300 produced by Tokyo Kasei Co., Ltd.) were placed in a 4.55 liter (1 gallon) polystyrene kneader (produced by Inoue Seisakusho o., Ltd.) and kneaded for 3 hours. The resulting mixture was poured into a 2.5 liter hot water, and stirred in a high speed mixer at about 80 °C for about one hour to obtain a slurry. The resulting slurry was filtered off, washed with water 5 times to eliminate the sodium chloride and the solvent, and dried employing a spray drying method. Thus, processed pigment was obtained.

### Epoxy compound

EP-A: Epoxy compound in which two epoxy-containing groups are connected through an ester group (see the following chemical structure).

EP-B: Epoxy compound in which two epoxy-containing groups are connected through an ester group (see the following chemical structure).

EP-C: Epoxy compound in which two epoxy-containing groups are connected through an ester group (see the following chemical structure). 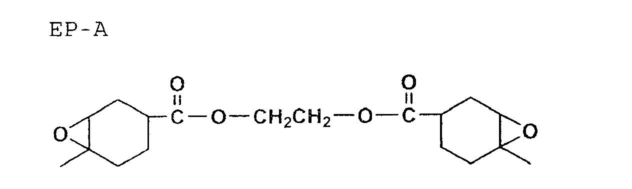 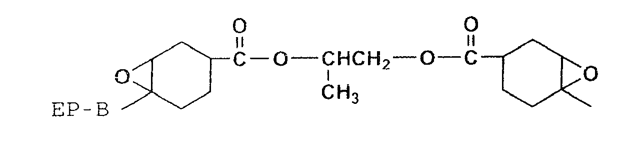 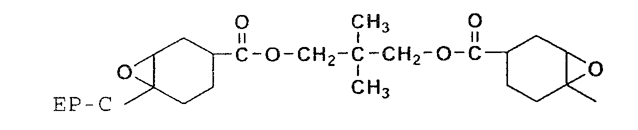 Celloxide 3000 (with a molecular weight of 168, produced by Daicel Kagaku Co., Ltd.)
Oxetane compound OXT221: Oxetane compound (produced by Toa Gosei Co., Ltd.)

### Vinyl ether compound DVE-3:

Triethylene glycol divinyl ether (produced by ISP Co., Ltd.) Cationic photopolymerization initiator:
SP-1: Triphenyl sulfonium salt (produced by Asahi Denka Co., Ltd.)
SP-2: Triphenyl sulfonium salt (produced by Asahi Denka Co., Ltd.)
SP-3: Triphenyl sulfonium salt (produced by Asahi Denka Co., Ltd.)

### <<Evaluation>>

The actinic energy radiation hardenable composition obtained above and the epoxy compound contained in the actinic energy radiation hardenable were evaluated as follows. (Safety of epoxy compound):

Irritation of the epoxy compound adhered to the skin was evaluated according to the following criteria:
A: No variation was observed on the skin.
B: The skin was colored red.
C: Blisters were produced on the skin.

### (Stability of actinic energy radiation hardenable composition)

The actinic energy radiation hardenable composition, after stored at 70 °C for one month, was observed and its viscosity was determined, and evaluated according to the following criteria:
A: Neither precipitation production nor viscosity variation was observed.
B: No precipitation production was observed, but an increase in viscosity was observed.
C: Precipitation production was observed.

### (Safety of actinic energy radiation hardenable composition)

Irritation of the actinic energy radiation hardenable composition adhered to the skin was evaluated according to the following criteria:
A: No variation was observed on the skin.
B: The skin was colored red.
C: Blisters were produced on the skin.

### (Hardenability)

The following six hardening methods were carried out under conditions as shown in Table 2, and hardenability was evaluated by minimum exposure energy at which tackiness of a hardened layer was not perceived by fingering. The lower the exposure energy (mJ/cm²) is, the better the hardenability.

### <Hardening method 1>

The actinic energy radiation hardenable composition was coated on a 0.8 mm thick, 50 mm wide and 150 mm long bondelight steel plate to form a layer with a thickness of 10 µm. The resulting sample was passed under a 80 W/cm concentrating high pressure mercury lamp, 10 cm distant from the lamp to obtain a hardened layer.

### <Hardening method 2>

Hardening method 2 was carried out in the same manner as Hardening method 1 above, except that a transparent polycarbonate plate was used instead of the bondelight steel plate.

### <Hardening method 3>

The actinic energy radiation hardenable composition was coated on a surface-treated biaxially stretched polypropylene film with a thickness of 30 µm in a coating amount of 1.0 g/m², employing a roll coater, and then a surface-treated unstretched polypropylene film with a thickness of 20 µm was pressure adhered onto the coated layer. The resulting sample was treated in the same manner as Hardening method 1 to obtain a hardened layer.

### <Hardening method 4>

Hardening method 4 was carried out in the same manner as Hardening method 1 above, except that an art paper was used instead of the bondelight steel plate.

### <Hardening method 5>

A solid shaped form was obtained employing a solid shaping system having a control section comprised mainly of a three dimensional NC (numerical control) provided with a vessel in which an actinic energy radiation hardenable composition was introduced, argon laser (with 333, 351 and 364 nm wavelengths), an optical system and a personal computer. Thus, a solid shaped form having a width of 100 mm, a length of 100 mm and a thickness of 10 mm in which a layer of the composition was laminated at a pitch of 0.1 mm, was obtained based on the dimension controlled according to CAD.

### <Hardening method 6>

The ink composition obtained was ejected from piezo type ink-jet nozzles ejecting ink droplets having a size of 7 p1 onto a corona-discharged polyethylene terephthalate film substrate, the nozzle portions being heated to 50 °C. In this case, the nozzle pitch was 360 dpi, ("dpi" representing the number of dots per inch or 2.54 cm). Thus, a solid image and 6-point MS Ming type characters were printed on the substrate. The resulting sample was exposed to a fluorescent tube having a main peak of 308 nm as a light source. Exposure was carried out to give an illumination intensity of 10 mW/cm² on the surface of the substrate placed immediately under the light source, wherein exposure was started 0.2 second after the ink was deposited on the substrate, and terminated 0.7 second later.

### (Layer strength)

A scratch test was carried out, in which a hardened layer hardened at 35 °C and 85% RH was scratched with fingernails, and strength of the hardened layer was evaluated according to the following criteria:
A: No hardened layer was peeled off by fingernail scratching.
B: A part of the hardened layer was peeled off by vigorous fingernail scratching.
C: The hardened layer was easily peeled off by fingernail scratching.

### (Solvent resistance and water resistance)

A sample prepared in the same manner as in layer strength evaluation above, after was immersed in 50 °C alcohol or in 50 °C water for 10 seconds, was observed for breakage and shrinkage of the image, and evaluated according to the following criteria:
A: No change was observed.
B: Slight breakage and shrinkage were observed.
C: Obvious breakage and shrinkage were observed.

The results are shown in Table 2.

**Table 2**

| Experiment No. | Actinic energy radiation hardenable composition No. | | | | | | | | | | |
|---|---|---|---|---|---|---|---|---|---|---|---|
| | No. | Safety of epoxy compound used | Safety | Stability | Layer hardened at 35 °C and 85% RH | | | Hardenability | | | |
| | | | | | | | | Hardening method No | Conditions | | |
| | | | | | Layer strength | Solvent resistance | Water resistance | | (i) | (ii) | (iii) |
| 1-1 | 1 (Inv.) | A | A | A | A | A | A | 1 | 50 | 50 | 100 |
| 1-2 | 2 (Inv.) | A | A | A | A | A | A | 2 | 50 | 50 | 100 |
| 1-3 | 3 (Inv.) | A | A | A | A | A | A | 3 | 50 | 50 | 100 |
| 1-4 | 4 (Inv.) | A | A | A | A | A | A | 4 | 50 | 50 | 100 |
| 1-5 | 5 (Inv.) | A | A | A | A | A | A | 5 | 50 | 50 | 100 |
| 1-6 | 1 (Inv.) | A | A | A | A | A | A | 6 | 50 | 50 | 100 |
| 1-7 | 6 (Comp.) | A | A | B | B | B | A | 1 | 50 | 50 | 100 |
| 1-8 | 7 (Comp.) | A | A | B | B | B | A | 2 | 50 | 50 | 100 |
| 1-9 | 8 (Comp.) | A | A | B | B | B | A | 3 | 50 | 50 | 100 |
| 1-10 | 9 (Comp.) | A | A | B | B | B | A | 4 | 50 | 50 | 100 |
| 1-11 | 6 (Comp.) | A | A | B | B | B | A | 6 | 50 | 50 | 100 |

| | | | | | | | | | | | |
|---|---|---|---|---|---|---|---|---|---|---|---|
| Inv.: Inventive, Comp.: Comparative (i) : 25°C and 45% RH (ii) : 25°C and 85% RH (iii) : 35°C and 85% RH | | | | | | | | | | | |

As is apparent from Table 2, the inventive actinic energy radiation hardenable compositions excel in stability, layer strength and solvent resistance as compared to comparative actinic energy radiation hardenable compositions.

The present invention provides an epoxy compound or an actinic energy radiation hardenable composition each having high safety and stability, and provides an actinic energy radiation hardenable composition with excellent photo-hardenability under high humidity, which gives high solvent resistance, high water proof and a hardened layer with high strength.

## Claims

1. A composition **characterized in that** it contains an epoxy compound represented by the following formula (1), 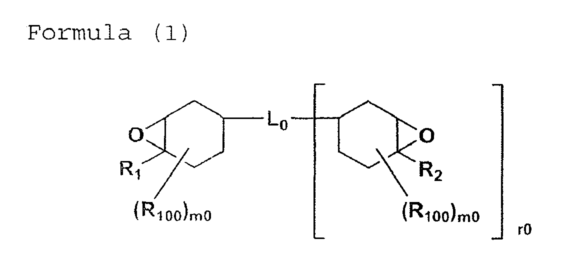 wherein R₁ and R₂ independently represent a first substituent (other than a hydrogen atom); R₁₀₀ represents a second substituent; m0 represents an integer of from 0 to 2; r0 represents an integer of from 1 to 3; and L₀ represents a simple bond or a (r0+1)-valent linkage group with a carbon atom number of from 1 to 15 containing in the main chain an oxygen atom and a nitrogen atom.

2. The composition of claim 1 **characterized in that** the epoxy compound is represented by the following formula (2), 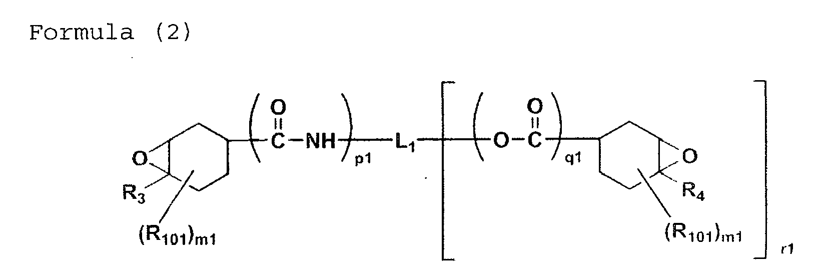 wherein R₃ and R₄ independently represent a third substituent (other than a hydrogen atom); R₁₀₁ represents a fourth substituent; m1 represents an integer of from 0 to 2; p1 represents an integer of from 1 to 2; q1 represents an integer of 0, 1 or 2; r1 represents an integer of 1 to 2; and L₁ represents a simple bond or a (r1+1)-valent linkage group with a carbon atom number of from 1 to 15 containing in the main chain an oxygen atom or a sulfur atom.

3. An actinic energy radiation hardenable composition **characterized in that** it contains an epoxy compound represented by the following formula (1), 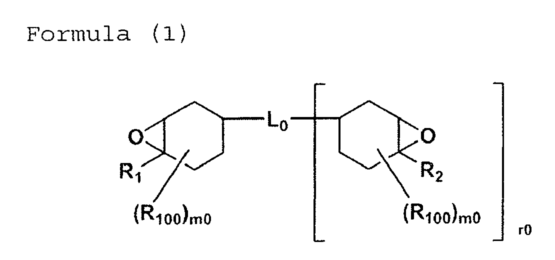 wherein R₁ and R₂ independently represent a first substituent (other than a hydrogen atom); R₁₀₀ represents a second substituent; m0 represents an integer of from 0 to 2; r0 represents an integer of from 1 to 3; and L₀ represents a simple bond or a (r0+1)-valent linkage group with a carbon atom number of from 1 to 15 containing in the main chain an oxygen atom and a nitrogen atom.

4. The actinic energy radiation hardenable composition of claim 3 **characterized in that** the epoxy compound is represented by the following formula (2), 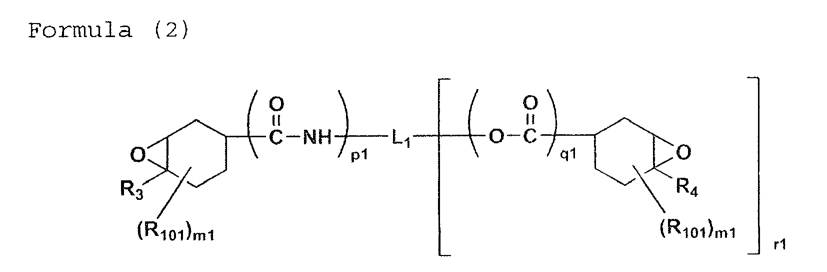 wherein R₃ and R₄ independently represent a third substituent (other than a hydrogen atom); R₁₀₁ represents a fourth substituent; m1 represents an integer of from 0 to 2; p1 represents an integer of from 1 to 2; q1 represents an integer of 0, 1 or 2; r1 represents an integer of from 1 to 2; and L₁ represents a simple bond or a (r1+1) -valent linkage group with a carbon atom number of from 1 to 15 containing in the main chain an oxygen atom or a sulfur atom.

5. The actinic energy radiation hardenable composition of claim 3 or 4 **characterized in that** the epoxy compound has a molecular weight of from 170 to 1000.

6. The actinic energy radiation hardenable composition of any one of claims 3 through 5 **characterized in that** it further contains an oxetane compound or a vinyl ether compound.

7. The actinic energy radiation hardenable composition of any one of claims 3 through 6 **characterized in that** it further contains a cationic photopolymerization initiator.

8. The actinic energy radiation hardenable composition of any one of claims 3 through 7 **characterized in that** it further contains, as a polymerizable compound, an oxetane compound and further contains, as a cationic photopolymerization initiator, at least one selected from sulfonium salts producing no benzene on actinic ray exposure represented by the formulae (4) through (7), 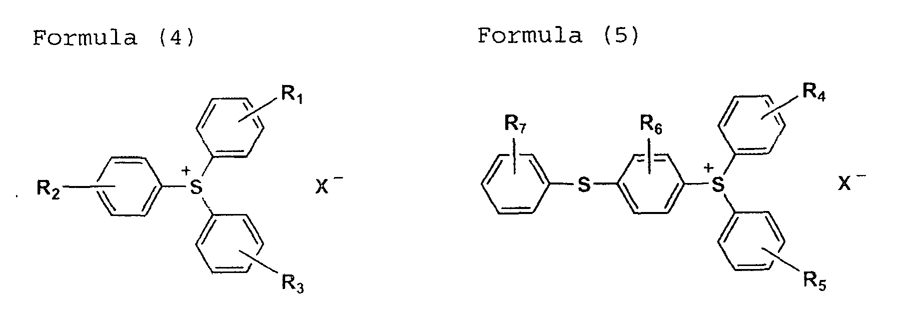 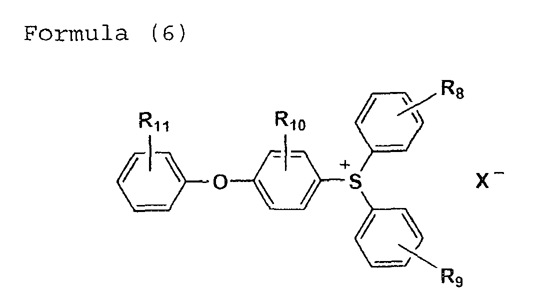 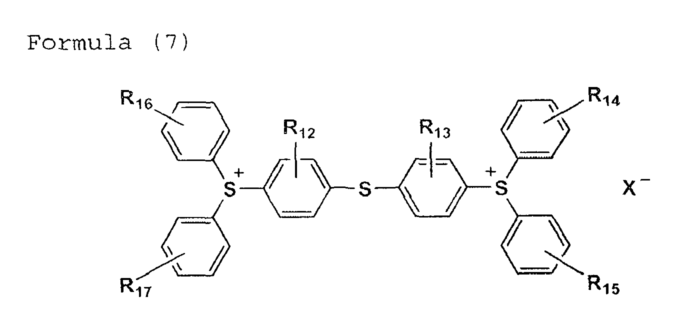 wherein R₁ through R₁₇ independently represent a hydrogen atom or a substituent, provided that R₁ through R₃ are not simultaneously hydrogens, R₄ through R₇ are not simultaneously hydrogens, R₈ through R₁₁ are not simultaneously hydrogens, and R₁₂ through R₁₇ are not simultaneously hydrogens; and X represents a non-nucleophilic anion.

9. The actinic energy radiation hardenable composition of claim 8 **characterized in that** the sulfonium salts represented by the formulae (4) through (7) include a sulfonium salt selected from the sulfonium salts represented by the formulae (8) through (16), 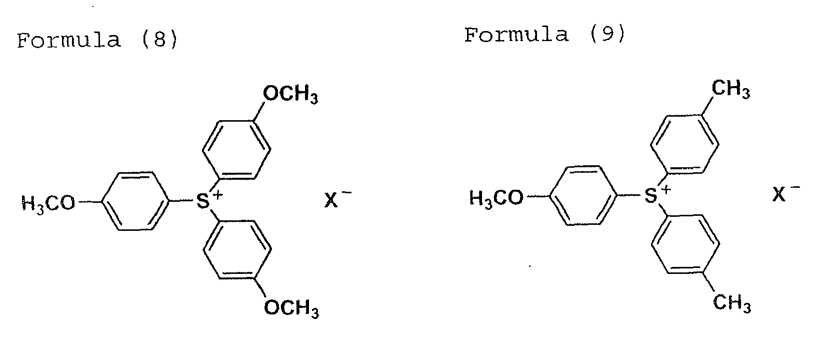 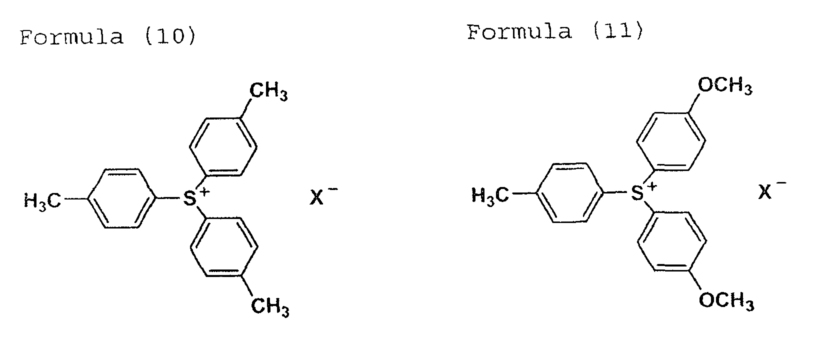 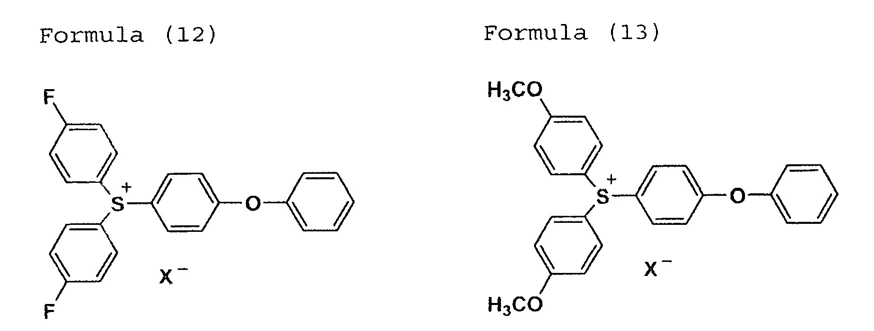 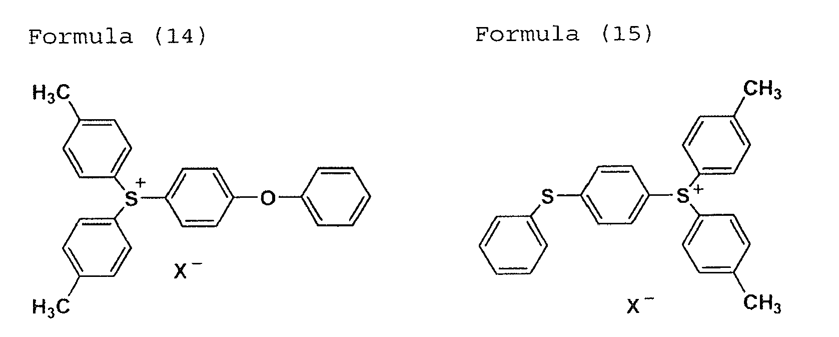 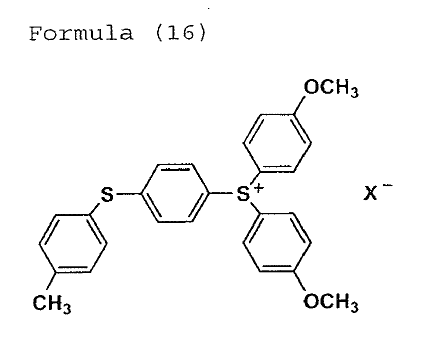 wherein x represents a non-nucleophilic anion.

10. The actinic energy radiation hardenable composition of any one of claims 3 through 9 **characterized in that** it further contains pigment.

11. An epoxy compound represented by the following formula (1), 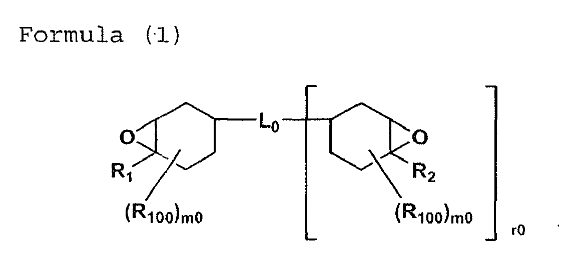 wherein R₁ and R₂ independently represent a first substituent (other than a hydrogen atom); R₁₀₀ represents a second substituent; m0 represents an integer of from 0 to 2; r0 represents an integer of from 1 to 3; and L₀ represents a simple bond or a (r0+1)-valent linkage group with a carbon atom number of from 1 to 15 containing in the main chain an oxygen atom and a nitrogen atom.

12. An epoxy compound represented by the following formula (2), 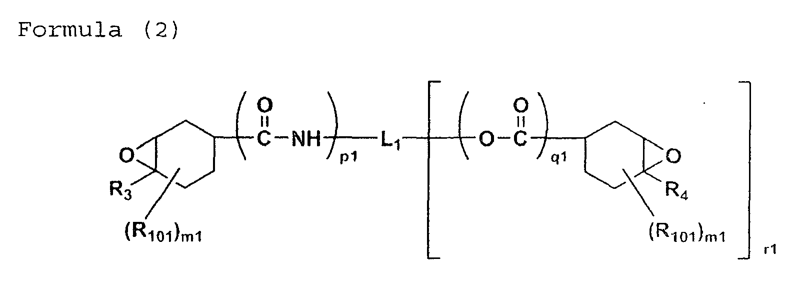 wherein R₃ and R₄ independently represent a third substituent (other than a hydrogen atom); R₁₀₁ represents a fourth substituent; m1 represents an integer of from 0 to 2; p1 represents an integer of from 1 to 2; q1 represents an integer of 0, 1 or 2; r1 represents an integer of from 1 to 2; and L₁ represents a simple bond or a (rl+l) -valent linkage group with a carbon atom number of from 1 to 15 containing in the main chain an oxygen atom or a sulfur atom

13. The epoxy compound of claim 11 or 12 **characterized in that** a value obtained by dividing the molecular weight with the total number of epoxy groups in the molecule is from 160 to 300.
